(19) Europäisches Patentamt
European Patent Office
Office européen des brevets

(11) Publication number: **0 057 736**
**B1**

# (12) EUROPEAN PATENT SPECIFICATION

(45) Date of publication of patent specification: **20.11.86**

(21) Application number: **81100833.3**

(22) Date of filing: **05.02.81**

(51) Int. Cl.$^4$: **C 07 C 6/04,** C 07 C 11/00, C 07 C 13/02, C 07 C 67/28, C 07 C 67/30, C 07 C 69/12, C 07 C 69/52, C 07 C 17/26, C 07 C 21/073, C 07 C 41/18, C 07 C 43/15

## (54) Olefin disproportionation process and catalyst.

(43) Date of publication of application:
**18.08.82 Bulletin 82/33**

(45) Publication of the grant of the patent:
**20.11.86 Bulletin 86/47**

(84) Designated Contracting States:
**BE FR NL**

(56) References cited:
**GB-A-1 524 163**
**US-A-3 689 433**

(73) Proprietor: **PHILLIPS PETROLEUM COMPANY**
**5th and Keeler**
**Bartlesville Oklahoma 74004 (US)**

(72) Inventor: **Banasiak, Dennis Stephen**
**3240 Jefferson Court**
**Bartlesville Oklahoma (US)**

(74) Representative: **Dost, Wolfgang, Dr.rer.nat., Dipl.-Chem. et al**
**Patent- und Rechtsanwälte Bardehle-Pagenberg-Dost-Altenburg & Partner Postfach 86 06 20**
**D-8000 München 86 (DE)**

**EP 0 057 736 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the disproportionation of olefins. More specifically, this invention relates to a novel catalyst system and a novel process for the disproportionation of olefins.

The disproportionation or metathesis of olefins is a reaction in which one or more olefinic compounds are transformed into other olefins of different molecular weights. The disproportionation of an olefin to produce one olefin of a higher molecular weight and one olefin of a lower molecular weight can also be referred to as a self-disproportionation. For example, propene can be disproportionated to ethylene and cis- and trans-2-butene. Another type of disproportionation involves the codisproportionation of two different olefins to form still other olefins. For example, the reaction of one molecule of 2-butene with one molecule of 3-hexene can produce two molecules of 2-pentene.

An especially valuable type of disproportionation involves the disproportionation of olefins bearing functional groups such as esters, ethers, chlorides. For example, 4-penten-1-yl acetate can be disproportionated to ethylene and cis- and trans-1,8-diacetoxy-4-octene. Likewise, 5-chloro-1-pentene can be disproportionated to ethylene and cis- and trans-1,8-dichloro-4-octene. The products bearing functional groups are valuable for use in polymer formation and chemical transformation to yield industrially valuable products. The term functional olefins is used herein to denote those olefins containing functionality other than hydrocarbyl olefinic unsaturation.

Several catalyst systems have been proposed for the disproportionation of olefins. Several disadvantages have been observed for these catalyst systems. In some cases, isomerization of the double bond of the starting material or product occurs and disproportionation involving the isomeric olefin yields a mixture of products which is difficult to separate. With certain catalysts, polymerization of the olefins occurs at long reaction times or high reaction temperatures. Some catalysts cause alkylation of aromatic solvents with the olefin, thereby consuming some of the reactant or product and producing a more complex product mixture. Some catalysts are only effective for terminal olefins and other catalysts may be effective only with internal olefins. Many of the metathesis catalyst systems used organoaluminum compounds which are expensive and present operational difficulties during production, storage, and use.

An object of the present invention is to provide a novel disproportionation catalyst which produces very little undesirable olefin isomerization and which does not require the employment of expensive organoaluminum components.

Another object of the present invention is to provide a process for reacting olefins to obtain non-polymeric disproportionation products. The term non-polymeric disproportionation products as used herein is intended to denote those products having molecular weights no greater than that of the sum of the molecular weights of four of the olefinic reactants.

Other objects, features and advantages of the present invention will appear more fully from the following description.

Accordingly, the invention relates to an olefin disproportionation process which comprises contacting olefin reactants, as hereinafter defined, with a disproportionation catalyst to cause a disproportionation of the olefin reactants into non-polymeric disproportionation products which process is characterized by conducting said disproportionation process in the presence of a catalyst composition comprising

(1) at least one inorganic metal compound selected from $SnCl_4$, $SnBr_4$, $GeCl_4$, $TiBr_4$, $ZrCl_4$, $TaCl_5$, $MoCl_5$, $MoOCl_4$, $ReCl_5$, $FeCl_3$, $CuCl_2$, $CuBr_2$, $WCl_6$ and $WOCl_4$; and

(2) at least one neutral carbene complex of the formula

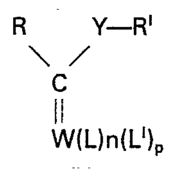

(I)

wherein

R is an aryl or substituted aryl radical containing 6 to 30 carbon atoms, the substituents in the case of substituted aryl being selected from halo and alkyl and alkoxy radicals of 1 to 20 carbon atoms;

$R^I$ is an alkyl, cycloalkyl, aryl, substituted aryl, triarylsilyl or trialkylsilyl radical containing up to 30 carbon atoms per radical, the substitutents in the case of substituted aryl being as above defined in connection with R; L is a neutral ligand molecule selected from CO, NO, $PCl_3$, $PF_3$, pyridine and $PR^I_3$ where $R^I$ is as defined above;

$L^I$ is a cyclopentadienyl radical; p is 0 or 1; n is 5 when p is 0, or 2 when p is 1; and Y is oxygen or sulfur; and wherein said olefin reactants contain from 3 to 40 carbon atoms and one or more non-conjugated carbon-carbon double bonds and at least one of said olefin reactants contains a maximum of two carbon-carbon double bonds and is either

(a) an acyclic olefin of the formula

$$R^{III}CH{=}CHR^{IV}$$

wherein $R^{III}$ and $R^{IV}$ are independently hydrogen or an alkyl, alkaryl or alkenyl radical of up to 18 carbon atoms; or

(b) a monocyclic olefin of the formula

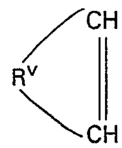

wherein $R^V$ is an alkylene or alkenylene radical containing from 5 to 16 carbon atoms wherein

— each of the radicals $R^{III}$ and $R^{IV}$ and $R^V$ may contain as substituents any of the following: halogen; an ester or ether group; an $—OSiR_3^{VI}$ or $—SiR_3^{VI}$ group where $R^{VI}$ is an alkyl, aryl or substituted aryl group of up to 20 carbon atoms, the substituents in the case of substituted aryl being as defined above in connection with R; $—N^+R_3{}^{VII}X^-$ where at least one $R^{VII}$ group is a $C_1—C_{10}$ alkyl group and the others are hydrogen or $C_1—C_{10}$ alkyl, and X is Cl, Br or I; such substituents in the case of halo, ester, ether and $—OSiR_3^{VI}$ groups being at least two carbon atoms distant from any doubly bound carbon atom in the olefin reactant and, in the case of $—SiR_3^{VI}$ and $—N^+R^{VII}{}_3\,X^-$ groups, at least one carbon atom distant from any such doubly bound carbon atoms; with the provisos that

— $R^I$ is other than aryl or substituted aryl when Y is sulfur, unless said inorganic metal compound is $SnCl_4$, $SnBr_4$ or $GeCl_4$ and also contains $SiCl_4$ or $SiBr_4$ as an additional component (and non-functional olefins are used); and

— Y is oxygen whenever the olefinic reactant contains an ester group as a substituent; and

— the metal component of the catalyst is a chloride or oxychloride when the olefin contains functional groups other than halogen.

Furthermore, the invention relates to an olefin disproportionation catalyst comprising an inorganic metal compound (1) and a neutral carbene complex (2) identified respectively as components (1) and (2) hereinbefore.

Furthermore, the invention relates to an olefin disproportionation catalyst comprising

(1) silicon tetrachloride;

(2) at least one neutral carbene complex of the formula

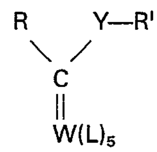

wherein R is an alkyl or cycloalkyl group of up to 10 carbon atoms or an aryl or substituted aryl radical of 6—30 carbon atoms, the substituent(s) in the latter case being selected from halogen and $C_1—C_{20}$ alkyl and alkoxy groups; $R^I$ and Y are as defined in claim 1; wherein R and $R^I$ are not both aryl or substituted aryl when Y is sulfur; and L is CO or NO; and

(3) carbon tetrachloride;

the molar ratio of (1):(2) being from 1:1 to 500:1 and the molar ratio of (3) to (1) being from 0.1:1 to 200:1.

In accordance with certain specific embodiments of the present invention, additional materials are added to the catalyst system to provide improved results. Many of the catalyst systems used in the present invention have the advantage of being effective for disproportionation of both functional and non-functional olefins.

With regard to the inorganic metal compound (1) mixtures of these metal compounds can be used if desired. An especially valuable mixture of metal compounds is tin tetrachloride and germanium tetrachloride which results in a higher yield than would be expected by the results of the individual halides.

With regard to the neutral carbene complex (2) for reasons of high reactivity, the currently preferred carbene complexes for use in the present invention are those where R is an aryl radical containing from 6 to 12 carbon atoms per radical or, when the metal halide component contains titanium, an alkyl radical containing 1 to 10 carbon atoms per radical, $R^I$ is an alkyl radical containing from 1 to 10 carbon atoms per radical, Y is O or S with the limitation that Y is not S when the metal compound is Ti, L is CO, n=5, and p=0.

For reasons of ease of preparation and high reactivity, the currently most preferred carbene complex is (methoxyphenylcarbene)pentacarbonyltungsten (O) which can be represented by the following formula II:

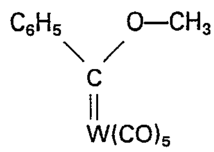

(II)

wherein $C_6H_5$ is a phenyl group.

The carbene complexes can be prepared by any of several published procedures, viz., D. J. Cardin et al, Chem. Rev., *72* 545 (1972), D. J. Cardin et al, Chem. Soc, Rev., *2*, 99 (1973), C. P. Casey, in "Transition Metal Organometallic in Organic Synthesis", Vol. 1, H. Alper, Ed., Academic Press, 1976, pp. 189—233; and "Inorganic Synthesis" Vol. 17, 95—99 (1979). In a typical preparation, carbene complex II is prepared by reacting tungsten hexacarbonyl with phenyllithium and then with trimethyloxonium tetrafluoroborate.

Specific examples of neutral carbene complexes include (methoxyphenylcarbene)pentacarbonyltungsten(O), (p-chlorophenylmethoxycarbene)pentacarbonyltungsten(O), (p-methylphenylmethoxycarbene)pentacarbonyltungsten(O), (p-methoxyphenylmethoxycarbene)pentacarbonyltungsten(O), (phenoxyphenylcarbene)pentacarbonyltungsten(O), (cyclohexyloxyphenylcarbene)pentacarbonyltungsten(O), (butoxyphenylcarbene)pentacarbonyltungsten(O), (octyloxyphenylcarbene)pentacarbonyltungsten(O), (hexadecyloxyphenylcarbene)pentacarbonyltungsten(O), (eicosyloxyphenylcarbene)pentacarbonyltungsten(O), (phenyltrimethylsilyloxycarbene)pentacarbonyltungsten(O), (phenyltriphenylsilyloxycarbene)pentacarbonyltungsten(O), (methylthiophenylcarbene)pentacarbonyltungsten(O), (dimethylaminophenylcarbene)pentacarbonyltungsten(O), (methoxyphenylcarbene)pentanitrosyltungsten(O), (methoxyphenylcarbene) $\eta^5$-cyclopentadienyldicarbonyl rhenium(1), (methoxyphenylcarbene)tetracarbonyl(triphenylphosphine)tungsten(O), and (methoxyphenylcarbene)pentacarbonylchromium(O). Mixtures of carbene complexes can be used if desired.

The ratio of the catalyst components can be expressed in terms of a molar ratio of metal compound to carbene complex. The molar ratio of the metal compound to the carbene complex in the present invention for Group IVa metal compounds, i.e., the germanium and tin compounds, is broadly from 1:1 to 500:1 and preferably from 5:1 to 100:1. For the metal compounds of Groups IVb, VIb, VIIb, and VIII, i.e., the compounds of titanium, zirconium, molybdenum, tungsten, rhenium and iron, the molar ratio of metal compound to carbene complex is broadly from 0.1:1 to 50:1 and preferably from 0.5:1 to 20:1.

For Group Vb and Ib metals, i.e., tantalum and copper, the molar ratio of metal compound to carbene complex is broadly from 0.1:1 to 8:1 and preferably from 0.5:1 to 5:1. At molar ratios lower than the lower limits, the disproportionation reaction is unacceptably slow while molar ratios above the upper limits result in no improvement or are detrimental to the reaction.

As mentioned above, the present invention also includes alternate embodiments wherein additional materials are employed in the catalyst system.

Embodiment A

When the metal compound comprises $GeCl_4$, $SnCl_4$ or $SnBr_4$, advantages are obtained by including silicon tetrachloride or tetrabromide as a promoter when in the carbene complex R, $R^I$, Y, $L^I$, p and n are as described hereinbefore in formula (I), with the limitation that R and $R^I$ are not both aryl or substituted aryl when Y is S and functional olefins are used, and L is CO or NO.

In this case the molar ratio of the germanium or tin compound to the carbene complex is broadly from 1:1 to 500:1 and preferably from 5:1 to 100:1. The molar ratio of silicon tetrahalide to the germanium or tin tetrahalide is broadly from 0.1:1 to 200:1 and preferably from 0.5:1 to 50:1.

Embodiment B

When the metal compound comprises tin tetrachloride, zirconium tetrachloride, tungsten hexachloride or tungsten oxychloride, advantages can be obtained by including a tetraorganogermanium having the formula $GeR^{II}_4$ wherein $R^{II}$ is an alkyl, aryl, or alkaryl radical containing from 1 to 10 carbon atoms per radical, when the carbene complex is as described in embodiment A. Examples of suitable tetraorganogermaniums include tetramethylgermanium, tetraethylgermanium, tetrapropylgermanium, tetrabutylgermanium, tetraoctylgermanium, tetradecylgermanium, tetraphenylgermanium, tetra-p-tolylgermanium and dimethyldiphenylgermanium.

The molar ratio of the tin tetrachloride to the carbene complex in this embodiment is broadly from 1:1 to 500:1 and preferably from 5:1 to 100:1. The molar ratio of the zirconium tetrachloride, tungsten hexachloride or tungsten oxychloride to the carbene complex is broadly from 0.1:1 to 50:1 and preferably from 0.5:1 to 20:1. The molar ratio of the tetraorganogermanium to the inorganic metal compound is broadly from 0.1:1 to 100:1 and preferably from 0.5:1 to 50:1.

Embodiment C

When the metal compound comprises a Group IVa metal tetrahalide, i.e., tin tetrachloride or tetrabromide, or germanium tetrachloride, advantages are provided by including carbon tetrachloride as a promoter when the carbene complex is as described for embodiment A.

In this embodiment the molar ratio of the Group IVa metal compound to the carbene complex is broadly from 1:1 to 500:1 and preferably from 5:1 to 100:1. The molar ratio of carbon tetrachloride to the Group IVa metal compound is broadly from 0.1:1 to 200:1 and preferably from 0.5:1 to 50:1.

As already indicated, an alternative aspect of the present invention resides in catalysts as described above in embodiment C but in which at least a part of the Group IVa metal compound is substituted by silicon tetrachloride.

Embodiment D

When the metal compound comprises zirconium tetrachloride advantages are obtained by including a tetraorganotin compound of the formula $SnR^{II}_4$ wherein $R^{II}$ is as described above in the discussion of embodiment B, when the carbene complex is as described for embodiment A.

The molar ratio of the zirconium tetrachloride to the carbene complex is broadly from 0.1:1 to 50:1 and preferably from 0.5:1 to 20:1. The molar ratio of the tetraorganotin promoter to the zirconium tetrachloride is broadly from 0.1:1 to 200:1 and preferably from 0.5:1 to 50:1.

Olefin reactants

The olefinic reactants have been identified hereinbefore. Generally the process involves the contacting of two olefinic reactants, which may be the same or different olefins. As indicated the olefins may be unsubstituted hydrocarbons, or they may contain functional substituents subject to certain limitations. For example, the first four of these functional groups, i.e., esters, ethers, halides, and trisubstituted silyloxy, must be located at least two saturated carbon atoms from the olefinic carbons, i.e., the functional group is on a carbon atom that is separated from the olefinic carbons by at least one saturated carbon atom. The last two functional groups must be located at least one carbon atom from the olefinic carbons. Moreover, for disproportionations involving olefins containing functional groups other than halogens, it appears that the metal compound component must be a chloride or oxychloride.

The olefinic double bond in an unsaturated ester can be located on either the alcohol or on the acid portion from which the ester was formally prepared. When an aryl group is present in the olefin, the aryl and olefinic groups should not be conjugated.

Examples of suitable acyclic olefinic reactants include propene, 1-butene, 1-pentene, 2-pentene, 1-hexene, 2-hexene, 1-octene, 2-octene, 4-methyl-1-heptene, 1-decene, 2-decene, 6-dodecene, 1-tetracene, 1-eicosene, 1,4-hexadiene, 4-phenyl-1-butene, 4-phenyl-1-octene, 4-pentene-1-yl acetate 4-chloro-1-pentene, 5-chloro-1-pentene, methyl oleate, methyl 10-undecenoate, ethyl oleyl ether, 3-butenyl methyl ether, 3-pentenyl propyl ether, methyl 4-hexenoate, ethyl 5-hexenoate, 4-bromo-1-butene, 4-iodo-1-butene, 3-buten-1-oxytrimethylsilane, 3-propen-1-yltrimethylsilane, and 3-butene-1-yltrimethylammonium chloride. Examples of suitable monocyclic olefins include cycloheptene, cyclooctene, cyclononene, cyclotetradecene, 4-chloro-1-cyclooctene, 1,5-cyclododecadiene, and 1,6-cyclodecadiene.

When two different olefinic reactants are utilized in the disproportionation, one of the olefins must be an olefin as described above and the other olefin can be either another olefin as described above or can be ethylene, a monocyclic monoolefin containing 4, 5 or 6 carbons in the ring, e.g., cyclobutene, cyclopentene, cyclohexene, or polycyclic mono- or diolefins. Examples of suitable polycyclic olefins include bicyclo[2.2.2]oct-2-ene, bicyclo[2.2.2]oct-2,5-diene, bicyclo[2.2.1]hept-2-ene, and bicyclo[3.3.0]oct-2-ene.

When two different olefinic reactants are employed in the disproportionation process, the molar ratio of one olefinic reactant to the other olefinic reactant is not critical, and, although about equimolar quantities are normally employed, up to as much as a 10-fold excess, preferably up to a 2-fold excess of one olefinic reactant can be employed.

The catalyst system of embodiment A is in addition useful for the disproportionation of the other of the above-recited functional olefins, i.e., those containing esters, ethers, trisubstituted silyloxy, trisubstituted silyl, and ammonium salts.

Reaction conditions

The amount of catalyst employed in the process of this invention can be expressed in terms of the molar ratio of olefin to carbene complex component. Generally, the molar ratio of the total stoichiometric amount of olefinic reactant to carbene complex component is in the range of 1:1 to 5000:1 and preferably from 50:1 to 2000:1. The term "total stoichiometric amount" is used herein to denote the moles of reactant that could theoretically react so as to distinguish from cases when two or more olefins are employed and one or more is used in a greater amount than will react. Thus, the amount of catalyst is generally based upon the amount of reactive olefins and not on excess olefin.

The disproportionation reaction of this invention can be carried out at temperatures between 35°C and 200°C. While lower temperatures can be used, the reaction rates are generally too low to be of interest. Temperatures above 200°C can be used, but excess decomposition of the reaction components can occur. The preferred reaction temperatures are from 50°C to 125°C.

The pressure during the disproportionation reaction can be from atmospheric to 5000 psig (34470 kPa). Preferably, the pressure is from atmospheric to 1000 psig (6894 kPa).

The disproportionation reaction can be carried out in the presence of diluents such as saturated hydrocarbons, e.g., hexane, octane, cyclohexane, aromatic hydrocarbons, e.g., benzene, toluene, or halogenated compounds, e.g., chlorobenzene, chloroform, methylene chloride or bromoform.

Diluents containing aromatic groups, e.g., chlorobenzene, have been found to sometimes undergo alkylation when utilized with Group VIb metal halides, e.g., $WCl_6$, at the higher metal compound component levels. This can of course result in decreased yields and therefore may be viewed as undesirable. Also the use of saturated hydrocarbons, e.g., hexane, as diluents with catalyst systems containing metal compounds from Group Vb frequently results in decreased yields and are thus less desirable. This indicates that the diluent is not a completely inert component of the reaction system and

attention must be paid to the diluent used for any specific combination of reaction ingredients. In embodiment C, the carbon tetrachloride component of the catalyst system can be used, especially in the upper portion of the concentration range, as a diluent.

The amount of diluent can be expressed as a volume ratio of diluent to the olefin. Suitable volume ratios of diluent to olefin can be from 0:1 to 500:1 and preferably from 1:1 to 300:1.

The presence of oxygen and water has been found to be deleterious to the disproportionation reaction and should be substantially avoided during the reaction. Inert gases such as nitrogen or helium can be used to maintain a dry, inert atmosphere during the reaction.

The metal compounds used as components of the catalyst system should be pure, free of oxygen or water, and free of any hydrolysis products. In general, the yield of disproportionation product decreases as the metal compound purity decreases.

The functional olefins used in the disproportionation reaction should be dry and free of polar materials such as carboxylic acids or alcohols. A purification step to remove impurities by such methods as filtering through silica gel and storing over molecular sieves or distilling from suitable drying agents is beneficial.

The reaction time period depends on the reaction temperature and pressure as well as on the nature of the particular catalyst system and olefinic reactant used. The reaction time is generally from 30 minutes to 14 days. Preferably the reaction time is from 2 to 120 hours. Olefins containing functional groups frequently undergo disproportionation reactions slower than non-functional olefins and longer reaction times may be appropriate.

Reaction product workup

The reaction product mixture from the disproportionation can be worked up using any combination of conventional separation and purification techniques. Depending on the relative volatilities of the unreacted starting olefins, the olefin products and the diluent, the reaction mixture can frequently be separated by fractional distillation. The unreacted starting olefin and diluent can be recycled to the reaction zone if desired. The olefin products can be purified by conventional techniques such as crystallization, distillation, or extractions.

The carbene catalyst can be removed, if desired, by the addition of dilute aqueous ammonia to decompose and precipitate the catalyst, followed by filtration and extraction. The resultant organic layer can then be worked up in a conventional manner.

Reaction products

According to the process of this invention, two olefinic reactants are disproportionated to form a product containing one or two olefins having a total number of carbon atoms equal to the sum of the carbon atoms of the two olefinic reactants and having a number of ethylenic double bonds equal to the sum of the ethylenic double bonds of the reactants.

One variation of the process comprises the disproportionation of two molecules of the same olefinic reactant. The reaction of two molecules of an acyclic olefin of formula VI generally produces one olefin of a higher carbon number and one olefin of a lower carbon number as depicted in equation (1)

$$2R^{III}CH=CHR^{IV} \rightarrow R^{III}CH=CHR^{III}+R^{IV}CH=CHR^{IV} \qquad (1)$$

wherein $R^{III}$ and $R^{IV}$ have the previously stated significance. If $R^{III}$ and $R^{IV}$ represent identical groups, it is appreciated that the disproportionation reaction will not cause any skeletal changes as the products $R^{III}CH=CHR^{III}$ and $R^{IV}CH=CHR^{IV}$ will be equivalent to $R^{IV}CH=CHR^{III}$. By way of specific illustration, the reaction of two molecules of propylene produces ethylene and 2-butene. However, the reaction of two molecules of 2-butene produces two molecules of 2-butene. When $R^{III}$ and $R^{IV}$ are combined to form a cyclic olefin, the reaction of two molecules of the cyclic olefin produces a single cyclic diolefin. At high dilution, the cyclic diolefin can be isolated, but in more concentrated solutions, further disproportionation frequently occurs to form materials of higher molecular weight. By way of specific illustration, the reaction of two molecules of cyclooctene in a dilute reaction mixture produces 1,9-cyclohexadecadiene.

Generally, when the disproportionation product contains an internal (non-terminal) double bond, a mixture of cis- and trans-isomers are present. For convenience, only one isomer is shown in the products below.

Another variation of the process comprises the disproportionation of two different acyclic olefinic reactants. By way of specific illustration, the reaction of 2-butene and 3-hexene produces two molecules of 2-pentene.

Still another variation of the process is "ring-opening" disproportionation wherein an acyclic olefinic reactant is contacted with a cyclic olefinic reactant. The product of "ring-opening" is a single olefinic compound with one less carbocyclic ring than the cyclic olefinic reactant, e.g.,

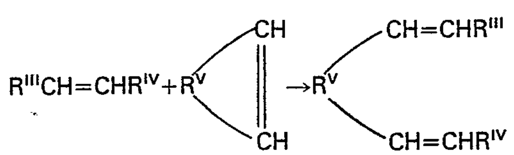

wherein $R^{III}$, $R^{IV}$, $R^{V}$ are as previously defined. By way of specific illustration, from reaction of 2-butene and cyclopentene is produced 2,7-nonadiene. Other typical products include 2,8-decadiene produced by reaction of cyclohexene and 2-butene, 3,8-undecadiene produced from 3-hexene and cyclopentene, 1,5,9-decatriene produced by reaction of ethylene and 1,5-cyclooctadiene, and 1,4-divinylcyclohexane from ethylene and bicyclo[2.2.2]oct-2-ene.

Olefins containing functional groups undergo disproportionation in a similar manner. For example, 4-penten-1-yl acetate yields a cis- and trans-mixture of 1,8-diacetoxy-4-octene and ethylene (equation 2)

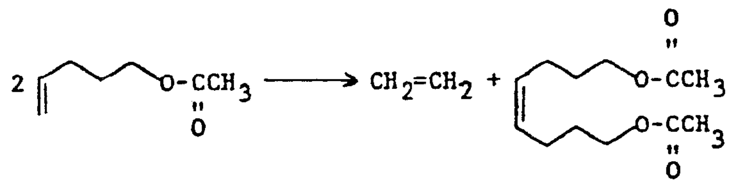

(2)

Methyl oleate disproportionates to cis- and trans- 9-octadecene and a cis- and trans-mixture of dimethyl 9-octadecen-1,18-dioate (equation 3).

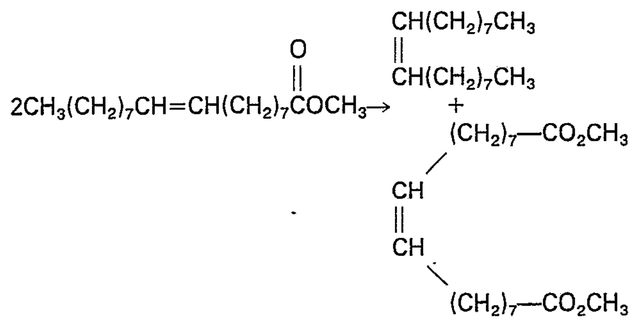

(3)

The co-disproportionation of cyclopentene with 4-penten-1-yl acetate yields a mixture of the co-disproportionation product 4,9-decadien-1-yl acetate and the self-disproportionation product from the 4-penten-1-yl acetate (both products contain cis- and trans-isomers).

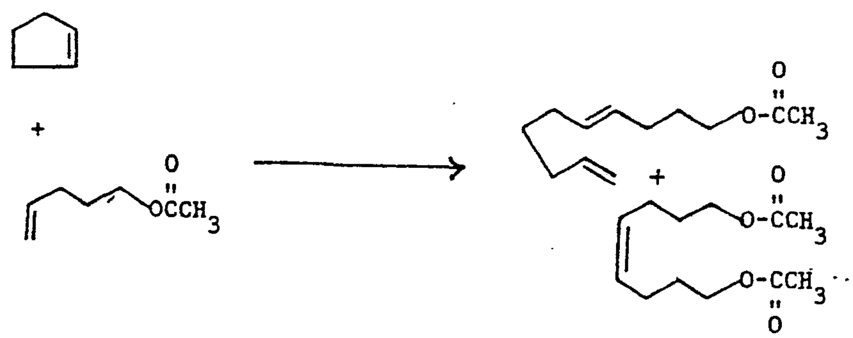

Some secondary disproportionation reactions between products and reactants are also frequently observed.

Product utility

The olefinic products, for the most part, have established utility as precursors of polymers, e.g., as the third component of ethylene-propylene terpolymers useful as synthetic elastomers. When functional groups are present on the olefinic product, the resulting polymer will contain functional groups which can be used to cross-link the polymer.

The olefinic products are converted to secondary and tertiary alcohols as by sulfuric acid-catalyzed hydration. Alternatively, the olefinic products are converted by conventional "Oxo" processes to aldehydes which are hydrogenated with conventional catalysts to the corresponding alcohols. The $C_{12}$—$C_{20}$ alcohols thereby produced are ethoxylated as by reaction with ethylene oxide in the presence of a basic catalyst, e.g., sodium hydroxide, to form conventional detergents and the lower molecular weight alcohols are esterified by reaction with polybasic acids, e.g., phthalic acid, to form plasticizers for polyvinyl chloride.

Olefinic products containing functional groups are useful as perfumes as well as for intermediates for the preparation of drugs and bioactive substances of insects.

Examples

The non-functional and functional olefins used in the following examples were commercially materials which were usually distilled, contacted with silica gel, and stored over 4A molecular sieves. Diluents were

usually distilled from the appropriate drying agent and stored over 4A molecular sieves. The various metal halides and organometallics were commercial materials which were normally used without further purification, but with careful exclusion of moisture and oxygen. In several cases, fresh reagents were found to give better disproportionation results that older reagents which are believed to have changed in chemical composition, for example by hydrolysis during aging. During the reaction evaluations, it was found that the careful exclusion of oxygen and moisture from the reaction system was extremely important for successful disproportionation results.

The carbene complexes were prepared by published procedures. Typically, they were prepared by reacting the appropriate metal carbonyl with an organolithium and then adding a trialkyloxonium tetrafluoroborate. For example, tungsten hexacarbonyl was reacted with phenyllithium and was then reacted with trimethyloxonium tetrafluoroborate to form (methoxyphenylcarbene)pentacarbonyltungsten(O)

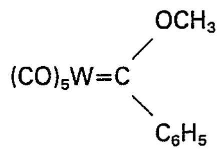

The introduction of alkylthio- or arylthio- groups was accomplished by reacting the corresponding alkoxy carbene complex with the appropriate thioalcohol, e.g.,

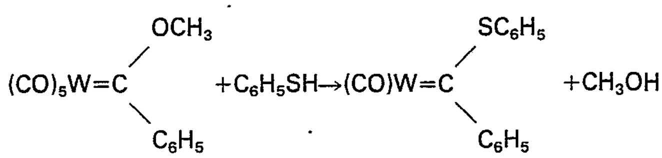

Trialkylsilyloxy groups were introduced by the reaction of the metal carbonyl with an organolithium, the addition of tetramethylammonium bromide, and reaction with a chlorotrialkylsilane. See E. O. Fischer et al, Chem. Ber., *110*, 2575—2583, 1977. (Methoxyphenylcarbene)tetracarbonyl(triphenylphosphine)-tungsten(O) was prepared by refluxing (methoxyphenylcarbonyl)pentacarbonyltungsten(O) with triphenylphosphine. The carbene complexes were stored in a desiccator in a freezer before use in a reaction.

Each of the runs in the following examples was carried out in a 10 oz. or 32 oz. (¼l or 1l) beverage bottle equipped with a magnetic stirrer, a self-sealing elastomeric cap liner, and a three-hole crown cap. The liquid reaction components were charged to the dried, nitrogen flushed bottle by syringe through the cap. Solids were added to the bottle before attaching the cap. The reaction mixture was heated to the desired temperature and stirred at the reaction temperature for the desired time period. At the conclusion of the reaction time period, the reaction mixture was analyzed by gas-liquid chromatography (glc). The glc peak areas were converted to weights using an internal standard. The disproportionation results are reported as a mole percent based on the conversion desired or accomplished. For example, since 2 molecules of 1-pentene are disproportionated to 1 molecule of ethylene and 1 molecule of 4-octene, the yields of 4-octene are expressed as a mole percent yield based on one half of the number of moles of 1-pentene charged to the reactor. The ethylene product was not determined and the 4-octene is a mixture of cis- and trans-isomers.

Reaction products containing internal double bonds were normally formed as a cis- and trans-mixture and, for simplicity, are not named cis- and trans-. The reaction products were identified in some instances by gas chromatograph/mass spectra and comparison of glc retention times with known materials. In many instances, the products were isolated from the reaction mixture and identified by elemental analysis, infrared spectra, nuclear magnetic resonance spectra, and mass spectra. The product isolation was carried out by diluting the reaction mixture with additional diluent, adding 10% aqueous ammonium hydroxide to precipitate the metal complexes, and filtering. The resulting filtrate was then processed by conventional techniques such as distillation or crystallization to yield the purified products.

Example I (Comparative)

Several control runs were carried out to demonstrate that the carbene complexes alone or the metal halides alone are not effective disproportionation catalysts. In run 1, 1.54 g (22 mmoles) of 1-pentene and 4.4 ml of a solution of (methoxyphenylcarbene)pentacarbonyltungsten(O) (0.11 mmoles) in chlorobenzene was charged to the reaction bottle. The mixture was stirred at room temperature (about 24°C) for 24 hours. A glc analysis indicated that no reaction had occurred. The mixture was heated to 130°C and stirred for 48 hours. Another glc analysis of the reaction mixture showed that no reaction had occurred.

In run 2, 1.54 g (22 mmoles) of 1-pentene, 4.4 ml of a solution of (methoxyphenylcarbene)pentacarbonyltungsten(O) (0.22 mmoles) in hexane, and 10 ml of hexane were charged to the reaction bottle.

The reaction mixture was stirred at 55°C for 4 days. A glc analysis of the reaction mixture showed that no reaction had occurred.

In run 3, 1.54 g (22 mmoles) of cis-2-pentene, 4.4 ml of a solution of (methoxyphenylcarbene)pentacarbonyltungsten(O) (0.11 mmoles) in chlorobenzene, and 2 ml of chlorobenzene were charged to the reaction bottle. The reaction mixture was stirred at 140°C for 20 hours. A glc analysis indicated that no reaction had occurred.

Two runs were carried out using tungsten hexachloride ($WCl_6$) and two different diluents. In each run, the reactor was charged with 1.54 g (22 mmoles) of 1-pentene, 0.22 mmoles of $WCl_6$, in run 4, 10 ml of hexane and in run 5 14.4 ml of chlorobenzene. The mixtures were stirred at 55°C for 20 hours (run 4) and 48 hours (run 5). The glc analysis indicated that no reaction had occurred in either run.

In run 6, the reaction bottle was charged with 22 mmoles of 1-pentene, 3 mmoles of tin tetrachloride, and 10 ml of chlorobenzene. At the conclusion of a 48 hour time period at 55°C with stirring, the glc analysis of the reaction mixture showed that no disproportionation had occurred.

The results of these runs show that neutral carbene complexes in the absence of activators are not effective for the disproportionation of olefins and that the metal halides alone are not effective catalysts for the disproportionation of olefins under these reaction conditions.

Example II

A series of runs was carried out involving the disproportionation of 1-pentene in the presence of a carbene complex and metal halides from Groups IVa, IVb, and Vb. In each run, the reaction bottle was charged with 22 mmoles of 1-pentene, 0.22 mmoles (0.44 mmoles in run 9) of (methoxyphenylcarbene)-pentacarbonyltungsten(O) in 4.4 ml of hexane or chlorobenzene, a metal halide, and additional hexane or chlorobenzene. The reagent quantities, reaction conditions, and the reaction results are presented in Table I.

TABLE I

| Run No. | Metal halide mmoles | | Group | Diluent, ml | Temp., °C | Time, hrs. | 4-Octene yield % |
|---|---|---|---|---|---|---|---|
| 7 | $SnCl_4$ | 6 | IVa | Hexane 15.4 | 55 | 24 | 52 |
| 8 | $SnCl_4$ | 2 | IVa | Hexane 8 | 55 | 22 | 37 |
| 9 | $SnCl_4$ | 4 | IVa | Hexane 20 | 55 | 96 | 20 |
| 10 | $SnBr_4$ | 2.7 | IVa | Hexane 15 | 55 | 120 | 37 |
| 11 | $GeCl_4$ | 2 | IVa | Hexane 9.4 | 150 | 20 | 10 |
| 17 | $TiBr_4$ | 3 | IVb | Hexane 14.4 | 55 | 24 | 5 |
| 18 | $ZrCl_4$ | 2.2 | IVb | Hexane 19.4 | 55 | 20 | 7 |
| 20 | $TaCl_5$ | 0.44 | Vb | $C_6H_5Cl$ 14.4 | 55 | 20 | 12 |

The results in Table I demonstrate the process of the present invention for the disproportionation of 1-pentene to 4-octene and ethylene in the presence of Group IVa, IVb, and Vb metal chlorides and bromides and a carbene complex. In a few of these runs (9, 10) at longer reaction times, a small amount of isomerization and halogenation of 1-pentene did occur during the reaction. In some instances, the 4-octene yields varied in repeated runs with the same metal halide, presumably due to further disproportionation at longer reaction times or to variations in catalyst purity.

Example III

Another series of runs was carried out involving the process of this invention for the disproportionation of 1-pentene in the presence of a carbene complex and metal compounds from Groups VIb, VIIb, VIII, and Ib. In each run, the reaction bottle was charged with 22 mmoles of 1-pentene, 0.22 mmoles of (methoxyphenylcarbene)pentacarbonyltungsten(O) in 4.4 ml of diluent (hexane or chlorobenzene), a metal halide or a metal oxyhalide, and additional diluent. Each run was carried out at 55°C. The reaction conditions and results are shown in Table II.

TABLE II

| Run No. | Metal halide mmoles | | Group | Diluent, ml | | Time, hrs. | 4-Octene yield % |
|---|---|---|---|---|---|---|---|
| 21 | $MoCl_5$ | 1 | VIb | $C_6H_5Cl$ | 34 | 24 | 42 |
| 22 | $MoCl_5$ | 0.33 | VIb | Hexane | 14.4 | 20 | 20 |
| 24 | $MoOCl_4$ | 0.44 | VIb | Hexane | 14.4 | 20 | 31 |
| 25 | $WCl_6$ | 0.22 | VIb | $C_6H_5Cl$ | 16.6 | 24 | 19 |
| 26 | $WCl_6$ | 0.22 | VIb | $C_6H_5Cl$ | 18.8 | 48 | 16 |
| 27 | $WCl_6$ | 0.22 | VIb | Hexane | 14.4 | 20 | 12 |
| 29 | $WOCl_4$ | 0.22 | VIb | Hexane | 16.6 | 24 | 3 |
| 30 | $WOCl_4$ | 0.44 | VIb | Hexane | 14.4 | 48 | 15 |
| 31 | $ReCl_5$ | 1 | VIIb | $C_6H_5Cl$ | 34 | 96 | 43 |
| 32 | $FeCl_3$ | 0.43 | VIII | Hexane | 14.4 | 20 | 26 |
| 33 | $CuCl_2$ | 0.22 | Ib | Hexane | 14.4 | 20 | 9 |
| 34 | $CuBr_2$ | 0.44 | Ib | Hexane | 14.4 | 72 | 30 |

The results presented in Table II illustrate the process of the present invention for the disproportionation of 1-pentene to 4-octene and ethylene in the presence of a carbene complex and a metal halide from Groups VIb, VIIb, VIII, and Ib or an oxyhalide of molybdenum and tungsten. Small amounts of isomerization and halogenation of the 1-pentene was observed in several of the runs.

Example IV

The use of mixtures of metal halides with carbene complexes for olefin disproportionation is illustrated in this example. The reaction bottle in each run was charged with 22 mmoles of 1-pentene, 0.22 mmoles of (methoxyphenylcarbene)pentacarbonyltungsten(O) in 4.4 ml of hexane, two different metal halides, and an additional quantity of hexane. The reactions were carried out of 55°C and the results and other ingredient quantities are shown in Table III.

TABLE III

| Run No. | Metal halide mmoles | | Metal halide mmoles | | Hexane ml | Time, hrs. | 4-Octene yield, % |
|---|---|---|---|---|---|---|---|
| 35 | $TiCl_4$ | 3 | $SnCl_4$ | 3 | 12.4 | 20 | 29 |
| 36 | $TiCl_4$ | 3 | $SnCl_4$ | 3 | 17.4 | 20 | 10 |
| 37 | $TiCl_4$ | 3 | $GeCl_4$ | 3 | 14.4 | 72 | 52 |
| 38 | $SnCl_4$ | 3 | $GeCl_4$ | 2 | 12.4 | 20 | 58 |

These results show that mixtures of metal halides can be used with a carbene complex for olefin disproportionation although the yields are sometimes below those obtained by the use of the individual metal halides. However, in run 38 using a combination of $SnCl_4$ and $GeCl_4$, a yield in excess of the expected yield from similar runs with the individual metal halides (see runs 8 and 11 from Table I) was obtained, suggesting a synergistic combination.

Example V

Several different olefins were contacted with the catalyst system of the present invention. In each run the olefin, (methoxyphenylcarbene)pentacarbonyltungsten(O), a metal halide, and a diluent were charged to the reaction bottle and the reactions were carried out at 55°C. The quantities utilized and the results of the runs are listed in Table IV.

TABLE IV

| Run No. | Metal halide, mmoles | | Olefin, mmoles | | Carbene complex mmoles | Diluent, ml | | Time, hrs. | Product yield % |
|---|---|---|---|---|---|---|---|---|---|
| 39 | $SnCl_4$ | 4 | 1-Hexene | 44 | 0.44 | Hexane | 20 | 96 | 30 |
| 40 | $SnCl_4$ | 40 | Cyclooctene | 200 | 2 | Hexane | 310 | 120 | [a] |
| 41 | $WCl_6$ | 2 | cis-2-Pentene | 22 | 0.22 | $C_6H_5Cl$ | 16.6 | 20 | 56 |
| 42 | $WCl_6$ | 1 | 1-Decene | 100 | 1 | $C_6H_5Cl$ | 80 | 288 | 10[b] |
| 44 | $SnCl_4$ | 2 | Styrene | 22 | 0.22 | Hexane | 11.4 | 20 | t[d] |

[a] 1,9-Cyclohexadecadiene was isolated and identified, but a yield was not determined.
[b] Isolated yield.
[d] trace of trans-stilbene.

The disproportionations of 1-hexene to 5-decene and ethylene, cyclooctene to 1,9-cyclohexadecadiene, cis-2-pentene to 3-hexene and 2-butene, and 1-decene to 9-octadecene and ethylene (the product yield in Table IV refers to the first product listed when two products are formed) did occur in the presence of the catalyst systems of the present invention. Isoprene (run 43), a conjugated diene and styrene (run 44), which contains an aromatic ring conjugated with the double bond, are not suitable olefins for self-disproportionation with the present catalyst system.

Example VI

Several runs were carried out with carbene complexes different than the carbene complex used in previous examples. In each run, 22 mmoles of 1-pentene, a metal halide, 0.22 mmoles of a carbene complex, and from about 9 to about 16 ml of hexane or chlorobenzene were charged to the reaction bottle and the mixture was stirred at 55°C. The ingredients and reaction yields are presented in Table V.

TABLE V

| Run No. | Metal halide, mmoles | | Carbene complex | Time, hrs. | 4-Octene yield, % |
|---|---|---|---|---|---|
| 45 | $SnCl_4$ | 2.2 | 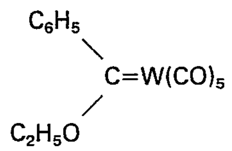 | 18 | 25 |
| 53 | $SnCl_4$ | 2 | 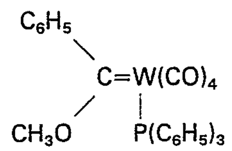 | 72 | 9 |
| 55 | $SnCl_4$ | 3 | 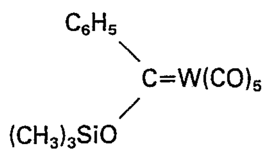 | 96 | 2 |
| 56 | $SnCl_4$ | 3 | 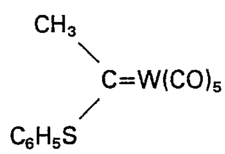 | 24 | 12 |

(Ethoxyphenylcarbene)pentacarbonyltungsten(O), run 45, (methoxyphenylcarbene)tetracarbonyl(triphenylphosphine)tungsten(O), run 53, (phenyltrimethylsilyloxycarbene)pentacarbonyltungsten(O), run 55, and (methylthiophenylcarbene)pentacarbonyltungsten(O), run 56, are shown in Table V to be suitable catalyst components for olefin disproportionation.

Example VII (Comparative)

Several runs were carried which show several changes in the components of the catalyst system of this invention which are detrimental to the disproportionation reaction. In each run, 22 mmoles of 1-pentene, 0.11, 0.2, or 0.22 mmoles of a carbene complex or a metal carbonyl, a second component, and 3 to 15 ml of hexane or chlorobenzene were charged to the reaction vessel. The quantities and reaction results are presented in Table VI.

TABLE VI

| Run No. | Metal halide or other, mmoles | | Carbene complex or metal carbonyl | Temp., °C | Time, hrs. | 4-Octene yield,[a)] % |
|---|---|---|---|---|---|---|
| 58 | $CuCl_2$ | 4.4 | 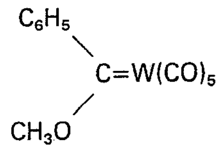 | 55 | 20 | 0 |
| 59 | $CuCl_2 \cdot 2H_2O$ | 0.44 | 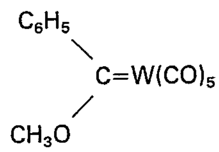 | 55 | 72 | 0 |
| 60 | $ReCl_3$ | 0.23 | 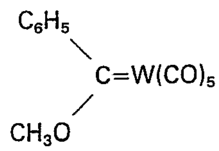 | 55 | 24 | 0 |
| 61 | $VCl_4$ | 2.2 | 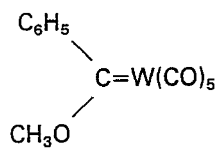 | 55 | 20 | 0 |
| 62 | $(CH_3CH_2)_3Al_2Cl_3$, | 2 | 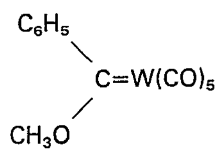 | 55 | 22 | t |
| 63 | $AlCl_3$ | 3 | 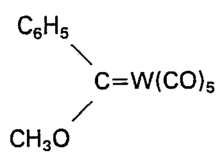 | 55 | 20 | 0 |
| 64 | $BCl_3$ | 2 | 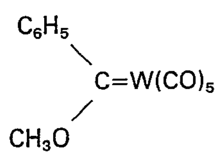 | 55 | 22 | 0 |
| 65 | $CH_3Li$ | 1.2 | 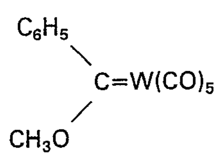 | 25 | 58 | 0 |
| 66 | $I_2$ | 0.44 | 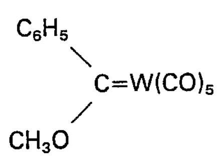 | 55 | 72 | 0 |
| 67 | $SnF_4$ | 2 | 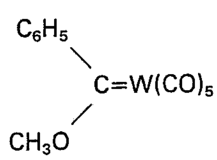 | 55 | 48 | 0 |

TABLE VI (contd.)

| Run No. | Metal halide or other, mmoles | | Carbene complex or metal carbonyl | Temp., °C | Time, hrs. | 4-Octene yield,[a] % |
|---|---|---|---|---|---|---|
| 68 | $SnCl_4$ | 2 | $W(CO)_6$ | 55 | 24 | 0 |
| 69 | $TiCl_4$ | 3 | $W(CO)_6$ | 55 | 20 | 0 |
| 70 | $WCl_6$ | 0.22 | 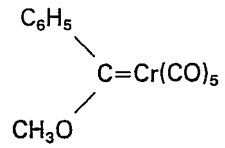 | 55 | 20 | 0 |
| 71 | $SnCl_4$ | 3 | 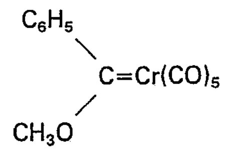 | 55 | 72 | t |
| 72 | $WCl_6$ | 0.22 | 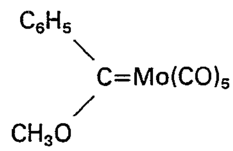 | 55 | 72 | 0 |
| 73 | $SnCl_4$ | 2 | 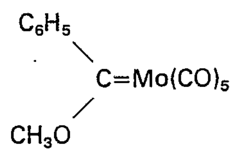 | 55 | 20 | t |
| 74 | $TiCl_4$ | 3 | 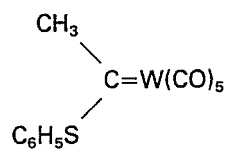 | 55 | 48 | 2 |
| 75 | $SnCl_4$ | 2.2 | 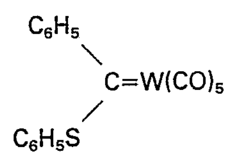 | 55 | 72 | 0 |

[a] t=trace

The use of $CuCl_2$ or $VCl_4$ at high levels (mole ratios of metal halide to carbene complex of 10/1 or more) in runs 58 and 61 were found to prevent disproportionation. The use of a metal chloride hydrate ($CuCl_2 \cdot 2H_2O$ in run 59) and rhenium in an oxidation state of 3 (run 60) were found to also prevent disproportionation. Several other materials in runs 62 through 67 were substituted for the metal compounds of the present invention and disproportionation did not occur. In runs 68 through 73, the tungsten carbene complex was replaced with $W(CO)_6$ or the corresponding chromium or molybdenum carbene complexes and essentially no disproportionation occurred. The use of a sulfur containing carbene complex with $TiCl_4$ in run 74 resulted in a yield significantly reduced from yields usually obtained with $TiCl_4$ (Table I). (Phenylthiophenylcarbene)pentacarbonyltungsten(O) and $SnCl_4$ did not result in a disproportionation of 1-pentene (run 75).

Example VIII

Runs are presented in this example to demonstrate embodiment A of the present invention. In embodiment A, the addition of a silicon tetrahalide to a carbene complex and a germanium, tin, or lead tetrahalide results in a more rapid disproportionation and higher yields. Each run was carried out as in the previous examples with 22 mmoles of 1-pentene and 0.22 mmoles of (methoxyphenylcarbene)penta-

carbonyltungsten(O). Runs 76 and 77 were carried out in a chlorobenzene diluent (16.6 ml) at 55°C. Samples were removed at intervals for glc analysis and the results are shown below.

| Time, hours | Run 76 2.2 mmoles $SnCl_4$ 4-octene, % | Run 77 2.2 mmoles $SnCl_4$ 3 mmoles $SiCl_4$ 4-octene, % |
|---|---|---|
| 2 | 3 | 8 |
| 5 | 22 | 22 |
| 7 | — | 36 |
| 8 | 24 | 45 |
| 24 | 35 | 40 |
| 48 | 37 | 34 |

The presence of $SiCl_4$ results in a more rapid conversion and a higher yield of 4-octene.

Runs 78 and 79 were carried out in a mixture of chlorobenzene (14.4 ml) and hexane (2.2 ml) at 55°C. The results are summarized below in terms of the yield of 4-octene at various times during the runs.

| Time, hours | Run 76 $SnCl_4$ (2.2 mmoles) 4-octene, % | Run 79 $SnCl_4$ (2.2 mmoles) $SiCl_4$ (3 mmoles) 4-octene, % |
|---|---|---|
| 2 | trace | — |
| 3 | — | 10 |
| 20 | 35 | 50 |
| 48 | 43 | — |
| 50 | — | 52 |

Again the presence of $SiCl_4$ increases the rate of reaction and yield of 4-octene.

Runs 80 through 83 were carried out in hexane (9 to 16 ml) at 55°C. The following results are expressed in terms of the yields of 4-octene at various times during the runs.

| Time, hours | Run 80 $SnCl_4$ (2.2 mmoles) 4-octene, % | Run 81 $SnCl_4$ (2 mmoles) $SiCl_4$ (3 mmoles) 4-octene, % |
|---|---|---|
| 1 | — | 20 |
| 1.5 | — | 45 |
| 2 | — | — |
| 3.3 | — | 58 |
| 4.3 | — | 70 |
| 5.5 | — | 64 |
| 6 | 9 | — |
| 7 | — | — |
| 8 | — | — |
| 21 | — | 61 |
| 24 | 31 | — |
| 31 | — | — |
| 48 | 65 | — |

| Time, hours | Run 82 $SnCl_4$ (2.2 mmoles) $SiCl_4$ (3 mmoles) 4-octene, % | Run 83 $SnCl_4$ (2.2 mmoles) $SiCl_4$ (3 mmoles) 4-octene, % |
|---|---|---|
| 1 | — | — |
| 1.5 | — | — |
| 2 | 1.6 | — |
| 3.3 | — | — |
| 4.3 | — | — |
| 5.5 | — | — |
| 6 | — | 13. |
| 7 | 26 | — |
| 8 | 39 | — |
| 21 | — | — |
| 31 | 66 | — |
| 48 | 65 | 66 |
| 24 | 62 | 64 |

While the results are variable, perhaps due to variations in reagent purity, the addition of $SiCl_4$ to the basic tin tetrachloridecarbene complex catalyst system results in a faster reaction and in a higher ultimate

yield. It is noted in the series of runs with various diluents in this example that hexane has a favorable influence on the product yields when $SiCl_4$ is used.

Several other runs (Table VII) were carried out at 55°C. The disproportionation of 1-pentene does not occur in the absence of the carbene complex (run 84) or in the absence of the $SnCl_4$ (run 85). In addition, the presence of diglyme as a diluent is detrimental to the reaction (run 86). The addition of $SiCl_4$ (run 87) to a catalyst system containing $GeCl_4$ and a carbene complex resultsd in an increase in yield compared with run 11 in Table I.

TABLE VII

| Run No. | Metal halide mmoles | | Other halide mmoles | | Diluent, ml | Time, hours | 4-Octene yield, % |
|---|---|---|---|---|---|---|---|
| 84[a)] | $SnCl_4$ | 2 | $SiCl_4$ | 3 | Hexane 5 | 48 | 0 |
| 85[b)] | None | | $SiCl_4$ | 20 | Hexane 8 | 24 | 0 |
| | | | | | Heptane 11 | | |
| 86[b)] | $SnCl_4$ | 2.2 | $SiCl_4$ | 3 | Diglyme 10 | 72 | 2 |
| | | | | | Hexane 6.6 | | |
| 87[b)] | $GeCl_4$ | 3 | $SiCl_4$ | 3 | Hexane 9.4 | 20 | 22 |

[a)] No carbene complex present.
[b)] 0.22 mmoles of (methoxyphenylcarbene)pentacarbonyltungsten(O).

Example IX

The use of embodiment A with several different carbene complexes or metal carbonyls for the disproportionation of 1-pentene is illustrated in this example. In each run, the reaction bottle was charged with 22 mmoles of 1-pentene, 0.2 to 0.3 mmoles of the carbene complex or metal carbonyl, $SnCl_4$, $SiCl_4$, and 9 to 18 ml of either hexane or chlorobenzene (runs 94 and 97) and the mixture was stirred at 55°C. The results are shown in Table VIII.

TABLE VIII

| Run No. | $SnCl_4$, mmoles | $SiCl_4$, mmoles | Carbene complex or metal carbonyl | Time, hours | 4-Octene yield, % |
|---|---|---|---|---|---|
| 88 | 2 | 3 | 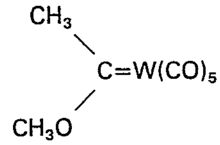 | 48 | 29 |
| 89 | 3 | 6 | 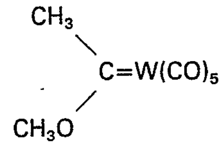 | 24 | 18 |
| 90 | 2.2 | 2 | 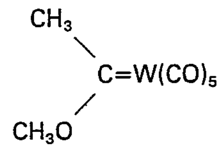 | 48 | 10 |
| 91 | 2.2 | 3 | 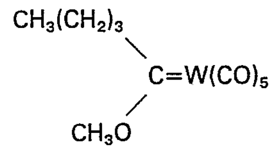 | 120 | 35 |
| 92 | 3 | 3 | 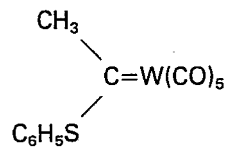 | 48 | 2 |
| 93 | 3 | 3 | 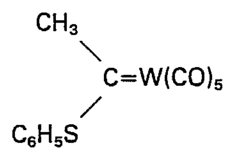 | 24 | 7 |
| 94 | 2.2 | 3 | 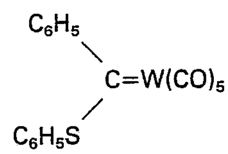 | 20 | 28 |
| 95 | 2.5 | 3 | 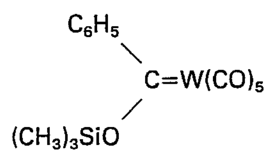 | 96 | 4 |
| 96 | 2 | 3 | $W(CO)_6$ | 48 | 0 |
| 97 | 0.3[a] | 3 | 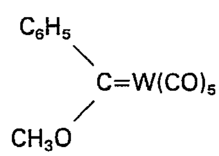 | 19 | 0 |

[a] $WCl_6$

The results in Table VIII show that a catalyst system containing a carbene complex, $SnCl_4$, and $SiCl_4$ is suitable for olefin disproportionation when alkyl groups (runs 88 to 91), thiophenyl (runs 92 to 94), or trimethylsilyloxy groups are present on the carbene carbon. The carbene complexes in runs 88 to 91 were shown previously (Example VI) to be ineffective as catalyst components with $SnCl_4$. Therefore, embodiment A also results in an increase in the range of carbene complexes suitable of olefin disproportionation. Run 96 indicates that $W(CO)_6$ cannot be successfully substituted for the carbene complex. The substitution of $WCl_6$ for the $SnCl_4$ (run 97) results in no disproportionation.

Example X

Several more runs were carried out to demonstrate the use of embodiment A of the present invention for the disproportionation of several different olefins. The reaction bottle in each run was charged with the olefin, $SnCl_4$, $SiCl_4$, (methoxyphenylcarbenepentacarbonyltungsten(O), and either hexane or chlorobenzene. The mixtures were stirred at 55°C. The quantities used and the results are presented in Table IX.

TABLE IX

| Run No. | $SnCl_4$, mmoles | $SiCl_4$, mmoles | Carbene complex mmoles | Olefin, mmoles | | Time, hours | Product yield, % |
|---|---|---|---|---|---|---|---|
| 98[a] | 2 | 3 | 0.22 | cis-2-Pentene | 22 | 48 | [c] |
| 99[b] | 10 | 15 | 1 | 1-Hexene | 1000 | 28 | 16[d] |
| 100[b] | 15 | 15 | 5 | 1-Hexene | 1000 | 48 | 23[d] |
| 101[b] | 20 | 30 | 2 | 1-Decene | 2000 | 120 | 12[d] |
| 102[b] | 50 | 50 | 5 | 1-Tetradecene | 500 | 48 | 30[d] |

[a] Chlorobenzene diluent
[b] Hexane diluent
[c] 3-Hexene is present but the yield could not be determined due to glc peak overlap.
[d] Isolated yield.

These runs illustrate the use of embodiment A for the disproportionation of cis-2-pentene to 3-hexene and 2-butene, 1-hexene to 5-decene and ethylene, 1-decene to 9-octadecene and ethylene, and 1-tetradecene to 12-hexacosene and ethylene. The yields in Table IX are isolated yields of the first of the two products listed above for each reaction.

Example XI

Embodiment B of the present invention is illustrated for the disproportionation of 1-pentene. This embodiment involves a catalyst system containing a carbene complex, a tin, zirconium, or tungsten halide or tungsten oxyhalide, and a tetraorganogermanium. In each run, 22 mmoles of 1-pentene, 0.22 mmoles of (methoxyphenylcarbene)pentacarbonyltungsten(O), several other components, and chlorobenzene or hexane (9 to 14 ml) were charged to the reactor and the mixtures were stirred at 55°C. The other components and the results are shown in Table X.

TABLE X

| Run No. | Metal halide, mmoles | Other, mmoles | Time, hours | 4-Octene yield, % |
|---|---|---|---|---|
| 103[a)] | None | $Ge(CH_3)_4$, 2 | 20 | 0 |
| 104[a)] | $SnCl_4$, 3 | $Ge(CH_3)_4$, 2 | 20 | 71 |
| 105[a)] | $SnCl_4$, 3 | $Si(CH_3)_4$, 2.2 | 20 | 45 |
| 106[a)] | $ZrCl_4$, 3 | $Ge(CH_3)_4$, 2 | 19 | 27 |
| 107[a)] | $ZrCl_4$, 3 | $Si(CH_3)_4$, 2.2 | 18 | 0 |
| 108[b)] | $WCl_6$, 0.22 | $Ge(CH_3)_4$, 2 | 48 | 30 |
| 109[b)] | $WCl_6$, 0.22 | $Ge(CH_3)_4$, 2 | 20 | 25 |

a) Hexane diluent.
b) Chlorobenzene diluent.

Tetramethylgermanium in combination with the carbene complex does not catalyze the disproportionation of 1-pentene (run 103). However, the presence of tetramethylgermanium with the carbene complex and $SnCl_4$ (run 104), $ZrCl_4$ (run 106), or $WCl_6$ (runs 108 and 109) results in higher yields of 4-octene than in runs in the absence of tetramethylgermanium (Tables I and II). Tetramethylsilane does not show the same beneficial results (runs 105 and 107).

Several control runs were carried out which show that the presence of tetramethylgermanium with a carbene complex and $TiCl_4$, $TiBr_4$, or $GeCl_4$ does not result in an improved yield over runs without the tetramethylgermanium. These runs were carried out as described above in this example for 20 hours at 55°C. The results are shown in Table XI.

TABLE XI

| Run No. | Metal halide, mmoles | Other, mmoles | Time, hours | 4-Octene yield, % |
|---|---|---|---|---|
| 110 | $TiCl_4$, 3 | $Ge(CH_3)_4$, 2 | 20 | 38 |
| 111 | $TiBr_4$, 3 | $Ge(CH_3)_4$, 2 | 20 | t[a)] |
| 112 | $GeCl_4$, 3 | $Ge(CH_3)_4$, 2 | 20 | 0 |

a) t=trace.

Example XII

The runs in this example involve carbon tetrachloride as a component of the catalyst system with carbene complexes and a Group IVa halide. Each run used 22 mmoles of 1-pentene, 0.22 mmoles of (methoxyphenylcarbene)pentacarbonyltungsten(O), various other materials, and hexane (9 to 14 ml) except in run 115 which did not include hexane. Each mixture was stirred at 55°C. The results are presented in Tables XII and XIII. Runs 85 and 11 are included from previous examples for comparison.

TABLE XII

| Run No. | Halide, compound mmoles | | Halide, mmoles | | Time, hours | 4-Octene yield, % |
|---|---|---|---|---|---|---|
| 85 | $SiCl_4$ | 20 | None | | 24 | 0 |
| 113 | None | | $CCl_4$ | 0.2 | 96 | 0 |
| 114 | None | | $CCl_4$ | 3 | 23 | 0 |
| 115 | None | | $CCl_4$ | 50 | 24 | 16 |
| 116 | $SiCl_4$ | 2 | $CCl_4$ | 3 | 24 | 31 |
| 11[a] | $GeCl_4$ | 2 | None | | 20 | 10 |
| 117 | $GeCl_4$ | 2 | $CCl_4$ | 3 | 20 | 27 |
| 118 | $SnCl_4$ | 2 | $CCl_4$ | 3 | 20 | 68 |
| 119 | $SnCl_4$ | 3 | $CCl_4$ | 3 | 72 | 56 |
| 120 | $SnCl_4$ | 3 | $CCl_4$ | 3 | 1 | t |
| | | | | | 2 | 9 |
| | | | | | 20 | 46 |
| 121 | $SnCl_4$ | 2 | $CCl_4$ | 3 | 3.3 | 19 |
| | | | | | 4.3 | 23 |
| | | | | | 21 | 57 |

[a] Reaction temperature=150°C.

TABLE XIII

| Run No. | Metal halide, mmoles | | Halide, mmoles | | Time, hours | 4-Octene yield, % |
|---|---|---|---|---|---|---|
| 122 | None | | $CBr_4$ | 3 | 20 | t |
| 123 | $SnCl_4$ | 3 | $CBr_4$ | 3 | 20 | 43 |
| 124 | None | | $Cl_3CCCl_3$ | 3 | 72 | 47 |
| 125 | $SnCl_4$ | 3 | $Cl_3CCCl_3$ | 3 | 48 | 62 |
| 126 | None | | $BrCCl_3$ | 3 | 20 | 13 |
| 127 | $SnCl_4$ | 3 | $BrCCl_3$ | 3 | 20 | 42 |
| 128 | None | | $Cl_3CC=CCl_2$<br>\|<br>Cl | 3 | 72 | 2 |
| 129 | $SnCl_4$ | 3 | $Cl_3CC=CCl_2$<br>\|<br>Cl | 3 | 20 | 9 |
| 130 | None | | O<br>‖<br>$Cl_3CCCl_3$ | 3 | 72 | 3 |
| 131 | $SnCl_4$ | 3 | O<br>‖<br>$Cl_3CCCCl_3$ | 3 | 20 | 3 |
| 132 | $GeCl_4$ | 3 | $Cl_3CC=CCl_2$<br>\|<br>Cl | 3 | 20 | 3 |
| 133 | $SiCl_4$ | 3 | $Cl_3CC=CCl_2$<br>\|<br>Cl | 3 | 19 | t |
| 134 | $SiCl_4$ | 3 | $Cl_3CCCl_3$ | 3 | 48 | 44 |
| 135 | $WCl_6$ | 0.22 | $CCl_4$ | 3 | 20 | 0 |

The results in Table XII show that $CCl_4$ in combination with a carbene complex results in olefin disproportionation only at the highest level (run 115). However, $CCl_4$ at low levels in combination with a carbene complex and $SiCl_4$, $GeCl_4$, or $SnCl_4$ result in higher disproportionation yields than runs in the absence of the $CCl_4$.

The control runs in Table XIII show that a variety of other halogenated materials do not give the improved disproportionation yield provided by $CCl_4$. Run 135 shows that $CCl_4$ is not suitable for improving the olefin disproportionation reaction with $WCl_6$ and a carbene complex.

Example XIII

This example illustrates embodiment D of the present invention for olefin disproportionation in the presence of a carbene complex, a zirconium halide, and a tetraorganotin. Each run was carried out in hexane (4 to 14 ml) at 55°C (except run 137) with 22 mmoles of 1-pentene, 0.22 mmoles of (methoxyphenylcarbene)pentacarbonyltungsten(O), and several other components. The results are presented in Table XIV.

TABLE XIV

| Run No. | Metal halide, mmoles | | $Sn(CH_3)_4$, mmoles | Time, hours | 4-Octene yield, % |
|---|---|---|---|---|---|
| 136 | None | | 2.2 | 96 | 0 |
| 137 | None | | 1.1 | a) | 0 |
| 138 | $ZrCl_4$ | 3 | 2 | 18 | 17 |
| 139 | $TiCl_4$ | 3 | 2 | 20 | 80 |
| 140 | $TiCl_4$ | 3 | 2 | 20 | 65 |
| 141 | $TiCl_4$ | 3 | 2 | 20 | 44 |
| 142 | $SnCl_4$ | 2 | 2 | 20 | 23 |
| 143 | $SnCl_4$ | 3 | 2 | 20 | 27 |
| 144 | $GeCl_4$ | 2 | 2 | 20 | 0 |
| 145 | $WCl_6$ | 0.22 | 2 | 20 | t[b)] |

[a)] 24 hours at room temperature
48 hours at 130°C
[b)] t=trace

These results show that although tetramethyltin in combination with a carbene complex (runs 136, 137) does not cause olefin disproportionation, the combination of tetramethyltin with a carbene complex and zirconium tetrachloride (run 138) does result in an improved disproportionation yield compared with a similar run in the absence of the tetramethyltin (run 18 of Table I). In contrast to this improvement, tetramethyltin results in either mixed results with $TiCl_4$ (runs 139 to 141) or reduced yields with $SnCl_4$ (runs 142 and 143), $GeCl_4$ (run 144), or $WCl_6$ (run 145) compared with runs in the absence of tetramethyltin (runs in Tables I and II).

Example XIV

The disproportionation of 4-penten-1-yl acetate to 1,8-diacetoxy-4-octene and ethylene in the presence of the catalyst system of this invention was demonstrated. In each run, the reaction bottle was charged with 16 mmoles of 4-penten-1-yl acetate (22 mmoles in run 155), (methoxyphenylcarbene)pentacarbonyl-tungsten(O), a metal halide, and either chlorobenzene or hexane (13 to 19 ml). The mixtures were stirred at 55°C. The quantities and results are shown in Table XV.

TABLE XV

| Run No. | Metal halide, mmoles | | Group | Carbene complex mmoles | Diluent | Time, hours | 1,8-Diacetoxy-4-octene yield,[a] % |
|---|---|---|---|---|---|---|---|
| 146 | $SnCl_4$ | 3.2 | IVa | 0.32 | $C_6H_5Cl$ | 48 | 33 |
| 147 | $SnCl_4$ | 3.2 | IVa | 0.16 | Hexane | 48 | 13 |
| 148 | $SnBr_4$ | 2 | IVa | 0.16 | $C_6H_5Cl$ | 120 | t |
| 149 | $WCl_6$ | 0.32 | VIb | 0.16 | $C_6H_5Cl$ | 120 | 7 |
| 150 | $WCl_6$ | 0.16 | VIb | 0.16 | $C_6H_5Cl$ | 72 | 5 |
| 151 | $WOCl_4$ | 0.32 | VIb | 0.16 | Hexane | 120 | 30 |
| 152 | $WBr_5$ | 0.44 | VIb | 0.16 | $C_6H_5Cl$ | 120 | 0 |
| 153 | $MoCl_5$ | 0.32 | VIb | 0.16 | Hexane | 168 | 3 |
| 154 | $MoOCl_4$ | 0.44 | VIb | 0.22 | Hexane | 120 | 2 |
| 155 | $TiCl_4$ | 3 | IVb | 0.22 | Hexane | 20 | 0 |
| 156 | $ReCl_5$ | 0.32 | VIb | 0.16 | $C_6H_5Cl$ | 20 | 1 |
| 157 | $SnF_4$ | 1.6 | IVa | 0.16 | $C_6H_5Cl$ | 48 | 0 |

[a] t=trace

These results demonstrate the process of this invention for the disproportionation of an unsaturated ester in the presence of $SnCl_4$, $WCl_6$, $WOCl_4$, $MoCl_5$, $MoOCl_4$, or $ReCl_5$ and a carbene complex. The metal bromides (runs 148 and 152) and fluorides (run 157) are ineffective as catalyst components. Run 155 suggests that $TiCl_4$ is not an effective catalyst component with esters.

Example XV

Several more runs were carried out with other unsaturated esters to demonstrate disproportionations with the catalyst system of the present invention. In the first group, methyl 10-undecenoate was disproportionated to ethylene and dimethyl 10-eicosen-1,20-dioate. In each run, 20 mmoles (50 mmoles in run 162) of methyl 10-undecenoate, 0.2 or 0.22 mmoles (0.5 mmoles in run 162) of (methoxyphenylcarbene)pentacarbonyltungsten(O), one or more metal chlorides, and chlorobenzene (hexane in run 161) 14 to 18 ml (25 ml in run 162 were utilized). All reactions were carried out at 55°C. The results in Table XVI are expressed in terms of the yield of dimethyl 10-eicosen-1,20-dioate.

TABLE XVI

| Run No. | Metal halide, mmoles | | Metal halide, mmoles | | Time, hours | Product yield,[a] % |
|---|---|---|---|---|---|---|
| 158 | $SnCl_4$ | 4 | None | | 48 | 46 |
| 159 | $SnCl_4$ | 2 | None | | 48 | 40 |
| 160 | $SnCl_4$ | 2 | None | | 20 | 22 |
| 161 | $SnCl_4$ | 2 | None | | 72 | 16 |
| 162 | $WCl_6$ | 0.5 | None | | 96 | 16 |
| 163 | $WOCl_4$ | 0.44 | None | | 72 | 5 |
| 164 | $SnCl_4$ | 0.22 | $WCl_6$ | 0.2 | 72 | 24 |
| 165 | $SnCl_4$ | 2 | $WCl_6$ | 0.1 | 72 | 20 |
| 166 | $SnCl_4$ | 2 | $WCl_6$ | 0.2 | 72 | 10 |
| 167 | $SnCl_4$ | 2 | $GeCl_4$ | 3 | 96 | 28 |

[a] Dimethyl 10-eicosen-1,20-dioate

Although the results in Table XVI are variable, they show that the combination of one or two metal chlorides or metal oxychlorides in combination with a carbene complex catalyzes the disproportionation of methyl 10-undecenoate.

The disproportionation of methyl oleate to 9-octadecene and dimethyl 9-octadecen-1,18-dioate was also carried out. Both runs used 22 mmoles of methyl oleate, 0.22 mmoles of (methoxyphenylcarbene)-pentacarbonyltungsten(O), $SnCl_4$ (2 or 2.2 mmoles), and 14.4 ml of chlorobenzene and were carried out at 55°C. In runs 168 and 169, the disproportionation of methyl oleate occurred. The yields are presented in Table XVII.

TABLE XVII

| Run No. | Metal halide, mmoles | | Time, hours | Diester yield,[a] % | 9-Octadecene yield, % |
|---|---|---|---|---|---|
| 168 | $SnCl_4$ | 2.2 | 20 | 3 | 4 |
| 169 | $SnCl_4$ | 2 | 72 | 11 | 11 |

[a] Dimethyl-9-octadecen-1,18-dioate

Example XVI

The co-disproportionation of cyclopentene with 4-penten-1-yl acetate in the presence of metal halides is demonstrated in this example. In each run, the reaction bottle was charged with cyclopentene (from 14 to 22 mmoles), 4-penten-1-yl acetate, (from 16 to 22 mmoles), a metal halide, (methoxyphenylcarbene)pentacarbonyltungsten(O), and chlorobenzene or hexane (6 to 24 ml). Each reaction was carried out at 55°C. Products identified from this reaction include 4,9-decadien-1-yl acetate (DDA), 1,8-diacetoxy-4-octene (DAO), and 4,9,14-pentadecatrien-1-yl acetate. The results are shown in Table XVIII.

TABLE XVIII

| Run No. | Metal halide, mmoles | | Carbene complex mmoles | Time, hours | DDA yield, % | DAO[a] yield, % |
|---|---|---|---|---|---|---|
| 170 | $SnCl_4$ | 1.6 | 0.16 | 96 | 15 | 16 |
| 171 | $SnCl_4$ | 4 | 0.44 | 72 | 17 | ND |
| 172 | $SnCl_4$ | 2 | 0.15 | 72 | 4 | ND |
| 173 | $WCl_6$ | 0.16 | 0.16 | 96 | 17 | 9 |
| 174 | $WCl_6$ | 0.22 | 0.22 | 20 | 14 | ND |
| 175 | $WCl_6$ | 1 | 0.22 | 24 | 10 | ND |
| 176 | $MoCl_5$ | 1 | 0.22 | 24 | 10 | ND |
| 177 | $MoBr_5$ | 0.22 | 0.22 | 48 | 0 | ND |
| 178 | $VCl_4$ | 2.2 | 0.22 | 20 | 0 | 0 |
| 179 | $ZrCl_4$ | 2.2 | 0.22 | 20 | 9 | ND |
| 180 | $TaCl_5$ | 0.44 | 0.22 | 20 | 14 | ND |
| 181 | $TaCl_5$ | 0.44 | 0.44 | 20 | 3 | ND |
| 182 | $ReCl_5$ | 1 | 0.22 | 20 | 14 | ND |
| 183 | $ReCl_5$ | 0.44 | 0.22 | 20 | 7 | 2 |
| 184 | $ReCl_5$ | 2.2 | 0.22 | 48 | 7 | ND |
| 185 | $FeCl_3$ | 0.43 | 0.22 | 20 | 2 | ND |

[a] ND=Not determined.

These results show that the combination of a carbene complex and a metal chloride are suitable for the co-disproportionation of cyclopentene with 4-penten-1-yl acetate. No disproportionation was observed with $MoBr_5$ or with $VCl_4$ at a level (mole ratio of $VCl_4$ to carbene complex of 10/1) above the range that is known to be effective.

Example XVII (Examples and Comparative Examples)

Several more runs were carried out to determine which functional groups can be successfully disproportionated with the catalyst system of present invention and the critical distances between the olefinic group and the functional group. In each run, the olefin (22 mmoles except in run 192 where 30 mmoles of each olefin was used), (methoxyphenylcarbene)pentacarbonyltungsten(O) (0.22 to 0.3 mmoles), a metal chloride, and either hexane or chlorobenzene (9 to 14 ml) were charged to the reaction bottle and the mixture was stirred at 55°C. Table XIX presents the results.

TABLE XIX

| Run No. | Metal halide, mmoles | | Olefin | Time, hours | Product yield, % |
|---|---|---|---|---|---|
| 186 | $SnCl_4$ | 2 | Allyl acetate | 20 | 0 |
| 187 | $SnCl_4$ | 2 | Allyl alcohol | 96 | 0 |
| 188 | $WCl_6$ | 0.22 | Allyl chloride | 48 | 0 |
| 189 | $SnCl_4$ | 0.22 | Allyloxytrimethylsilane | 24 | 0 |
| 190 | $TiCl_4$ | 3 | Allyloxytrimethylsilane | 20 | 0 |
| 191 | $SnCl_4$ | 3 | Cyclopentene and 3-butene nitrile | 24 | 0 |
| 192 | $WCl_6$ | 0.22 | 5-Hexen-2-one | 72 | 0 |
| 193 | $SnCl_4$ | 2.2 | 5-Chloro-1-pentene | 24<br>48 | 17[a)]<br>26[a)] |
| 194 | $SnCl_4$ | 4 | Ethyl oleyl ether | 20 | 10[b)]<br>11[c)] |

[a)] 1,8-Dichloro-4-octene
[b)] 9-Octadecene
[c)] 1,18-Diethoxy-9-octadecene

These results show that olefins containing ester (run 186), alcohol (run 187), chloride (run 188), trimethylsilyloxy (runs 189 and 190), or nitrile (run 191) groups located only one carbon atom from the olefinic group do not undergo disproportionation with the catalyst system of the present invention. Unsaturated ketones (run 192) are not suitable as disproportionation substrates. Unsaturated chlorides (run 193) and unsaturated ethers (run 194) with at least two carbon atoms between the olefinic group and the chloride or ether were found to undergo disproportionation in the presence of the catalyst system of this invention.

The results in this example show clearly the difference in behavior between the prior art catalyst systems and the catalyst system of this invention. For example, the disproportionation catalyst of U.S. 3,974,196 was capable of catalyzing the disproportionation of allyl acetate, 3-butenenitrile, and 5-hexen-2-one, while the present catalyst system was shown in Table XIX to be essentially inert towards these compounds. Therefore, the present catalyst system is more selective towards functional olefins than prior art catalysts.

Example XVIII

Embodiment A of this invention can be utilized for the disproportionation of unsaturated esters. Embodiment A uses a carbene complex, a germanium, tin, or lead tetrahalide, and silicon tetrahalide as the catalyst system. Table XX presents the results from runs in which 4-penten-1-yl acetate (16 mmoles) was disproportionated to ethylene and 1,8-diacetoxy-4-octene in the presence of (methoxyphenylcarbene)-pentacarbonyltungsten(O), $SnCl_4$, $SiCl_4$ and a diluent at 55°C.

TABLE XX

| Run No. | $SnCl_4$, mmoles | $SiCl_4$, mmoles | Carbene complex, mmoles | Diluent | Time, hours | Product yield,[a] % |
|---|---|---|---|---|---|---|
| 195 | 3.2 | 6 | 0.32 | $C_6H_5Cl$ | 24 | 42 |
| 196 | 1.6 | 3 | 0.16 | $C_6H_5Cl$ | 120 | 42 |
| 197 | 1.6 | 3 | 0.16 | Hexane | 48 | 33 |
| 198 | 1.6 | 3 | 0.16 | $C_6H_5Cl$ | 1 | 0.3 |
| | | | | | 2 | 1 |
| | | | | | 5 | 14 |
| | | | | | 24 | 40 |
| | | | | | 48 | 39 |
| 199 | 1.6 | 3 | 0.16 | $C_6H_5NO_2$ | 18 | 0 |
| 200 | 1.6 | 3 | 0.16 | $CH_3CN$ | 20 | 0 |

[a] 1,8-Diacetoxy-4-octene

The yields of 1,8-diacetoxy-4-octene were increased when compared with the yields in similar runs in Example XIV in the absence of $SiCl_4$. The use of nitrobenzene or acetonitrile as diluents (runs 199 and 200) was found to prevent disproportionation.

Example XIX

Several other runs were carried out to demonstrate the use of embodiment A for the disproportionation of unsaturated esters. In run 201, 21 mmoles of methyl oleate, 0.22 mmoles of (methoxyphenylcarbenepentacarbonyltungsten(O), 2.2 mmoles of $SnCl_4$, and 2.2 mmoles of $SiCl_4$ in about 18 ml of chlorobenzene were stirred at 55°C for 72 hours. The reaction product mixture contained a 16% yield of 9-octadecene and about a 10% yield of dimethyl 9-octadecen-1,18-dioate. The yield of 9-octadecene is increased over the yield of run 168 which did not use $SiCl_4$.

In run 202, methyl oleate and ethylene were co-disproportionated by contacting 22 mmoles of methyl oleate with ethylene (30 psi), 0.22 mmoles of (methoxyphenylcarbene)pentacarbonyltungsten(O), 2.2 mmoles of $SnCl_4$, and 3 mmoles of $SiCl_4$ in about 16 ml of hexane at 55°C for 72 hours. An analysis of the reaction product mixture showed that 1-decene and methyl 9-decen-1-oate were present in yield of 13% each.

Numerous runs were carried out to investigate the disproportionation of methyl 10-undecenoate to ethylene and dimethyl 10-eicosen-1,20-dioate using embodiment A of this invention. While the yield varied considerably from run to run, the average yield of dimethyl 10-eicosen-1,20-dioate from 7 runs using a mixture of methyl 10-undecenoate, (methoxyphenylcarbene)pentacarbonyltungsten(O), $SnCl_4$, and $SiCl_4$ in a mole ratio of 100/1/10/15 in chlorobenzene at 55°C for 3 to 6 days was 27%. The average yield of dimethyl 10-eicosen-1,20-dioate in a series of 6 runs using the same reagents and conditions except for the absence of $SiCl_4$ was 23%. Therefore, the presence of $SiCl_4$ increases the disproportionation product yield.

Example XX

Embodiment A of this invention was utilized with several other functional olefins. In each run, 20 to 22 mmoles of the olefin, 0.2 or 0.22 mmoles of (methoxyphenylcarbene)pentacarbonyltungsten(O), $SnCl_4$, and $SiCl_4$ in hexane or chlorobenzene were stirred at 55°C. The reaction product mixtures were analyzed by glc and the results are presented in Table XXI.

TABLE XXI

| Run No. | $SnCl_4$, mmoles | $SiCl_4$, mmoles | Olefin | Time, hours | Metathesis product yield, % |
|---|---|---|---|---|---|
| 203 | 2 | 3 | 6-Chloro-1-hexene | 48 | 60[a)] |
| 204 | 2.2 | 3 | 5-Chloro-1-pentene | 96 | 49[b)] |
| 205 | 2 | 3 | 4-Chloro-1-butene | 48 | 12[c)] |
| 206 | 2 | 4 | Ethyl oleyl ether | 96 | 9[d)] |
| | | | | | 12[e)] |
| 207 | 2.2 | 3 | Allyl chloride | 48 | 0 |
| 208 | 2.2 | 3 | Allyl acetate | 20 | 0 |
| 209 | 2 | 3 | Allyl ethyl ether | 96 | 0 |

a) 1,10-Dichloro-5-decene
b) 1,8-Dichloro-4-octene
c) 1,6-Dichloro-3-hexene
d) 9-Octadecene
e) 1,18-Ethoxy-9-octadecene

The catalyst system used in this example caused the disproportionation of a series of unsaturated chlorides with decreasing yields as the distance between the chloride and the olefinic group decreased (runs 203, 204, 205). The yield in run 204 was significantly increased over the corresponding run (run 193 of Example XVII) without the presence of $SiCl_4$. Ethyl oleyl ether (run 206) was disproportionated in slightly higher yields than the corresponding run (run 194 in Example XVII) in the absence of $SiCl_4$. Functional olefins having a chloride, ester, or ether located only one carbon atom from the olefinic group were inactive for disproportionation (runs 207 to 209).

Attempts to use (phenylthiophenylcarbene)pentacarbonyltungsten(O) in the catalyst system of embodiment A with several unsaturated esters yielded essentially no disproportionation. This suggests that this carbene complex is not suitable for use in embodiment A for functional olefins under the reaction conditions used. Also found to be unsuitable for use in embodiment A were (methoxymethylcarbene)-pentacarbonyltungsten(O), (methoxyphenylcarbene)pentacarbonylchromium(O), and (methylthiophenyl-carbene)pentacarbonyltungsten(O).

**Claims**

1. An olefin disproportionation process which comprises contacting olefin reactants, as hereinafter defined, with a disproportionation catalyst to cause a disproportionation of the olefin reactants into non-polymeric disproportionation products, characterized by conducting said disproportionation process in the presence of a catalyst composition comprising

(1) at least one inorganic metal compound selected from $SnCl_4$, $SnBr_4$, $GeCl_4$, $TiBr_4$, $ZrCl_4$, $TaCl_5$, $MoCl_5$, $MoOCl_4$, $ReCl_5$, $FeCl_3$, $CuCl_2$, $CuBr_2$, $WCl_6$ and $WOCl_4$; and

(2) at least one neutral carbene complex of the formula

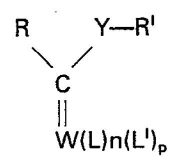

wherein

R is an aryl or substituted aryl radical containing 6 to 30 carbon atoms, the substituents in the case of substituted aryl being selected from halo and alkyl and alkoxy radicals of 1 to 20 carbon atoms;

$R^I$ is an alkyl, cycloalkyl, aryl, substituted aryl, triarylsilyl or trialkylsilyl radical containing up to 30 carbon atoms per radical, the substituents in the case of substituted aryl being as above defined in connection with R; L is a neutral ligand molecule selected from CO, NO, $PCl_3$, $PF_3$, pyridine and $PR^I_3$ where $R^I$ is as defined above;

$L^I$ is a cyclopentadienyl radical; p is 0 or 1; n is 5 when p is 0, or 2 when p is 1; and Y is oxygen or sulfur; and wherein said olefin reactants contain from 3 to 40 carbon atoms and one or more non-conjugated carbon-carbon double bonds and at least one of said olefin reactants contains a maximum of two carbon-carbon double bonds and is either

(a) an acyclic olefin of the formula

$$R^{III}CH{=}CHR^{IV}$$

wherein $R^{III}$ and $R^{IV}$ are independently hydrogen or an alkyl, alkaryl or alkenyl radical of up to 18 carbon atoms; or

(b) a monocyclic olefin of the formula

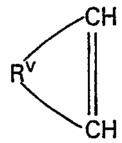

wherein $R^V$ is an alkylene or alkenylene radical containing from 5 to 16 carbon atoms wherein

— each of the radicals $R^{III}$ and $R^{IV}$ and $R^V$ may contain as substituents any of the following: halogen; an ester or ether group; an $—OSiR_3^{VI}$ or $—SiR_3^{VI}$ group where $R^{VI}$ is an alkyl, aryl or substituted aryl group of up to 20 carbon atoms, the substituents in the case of substituted aryl being as defined above in connection with R; $—N^+R_3^{VII}X^-$ where at least one $R^{VII}$ group is a $C_1—C_{10}$ alkyl group and the others are hydrogen or $C_1—C_{10}$ alkyl, and X is Cl, Br or I; such substituents in the case of halo, ester, ether and $—OSiR_3^{VI}$ groups being at least two carbon atoms distant from any doubly bound carbon atom in the olefin reactant and, in the case of $—SiR_3^{VI}$ and $—N^+—R^{VII}{}_3X^-$ groups, at least one carbon atom distant from any such doubly bound carbon atoms; with the provisos that

— $R^I$ is other than aryl or substituted aryl when Y is sulfur, unless said inorganic metal compound is $SnCl_4$, $SnBr_4$ or $GeCl_4$ and also contains $SiCl_4$ or $SiBr_4$ as an additional component and non-functional olefins are used and

— Y is oxygen whenever the olefinic reactant contains an ester group as a substituent; and

— the metal component of the catalyst is a chloride or oxychloride when the olefin contains functional groups other than halogen.

2. The process of claim 1 characterized in that said disproportionation is carried out at a temperature in the range of 35 to 200°C and under a pressure of from atmospheric to 34.5 MPa.

3. The process of claim 1 or 2 characterized in that the molar ratio of components (1) and (2) in the catalyst is from 1:1 to 500:1 when (1) is a group IVa metal compound; from 0.1:1 to 50:1 when (1) is a group IVb, VIb, VIIb or VIII metal compound; and from 0.1:1 to 8:1 when (1) is a group Vb or Ib metal compound.

4. The process of any of the preceding claims characterized in that R is an aryl radical containing 6 to 12 carbon atoms, $R^I$ is an alkyl radical containing 1 to 10 carbon atoms; L is CO, n is 5, and p is 0.

5. The process of claim 4, characterized in that said inorganic metal compound (1) contains Ti and Y is oxygen.

6. The process of any of the preceding claims characterized in that the olefin reactants are hydrocarbons.

7. The process of any of the preceding claims characterized in that the molar ratio of olefin reactant to carbene complex (2) is in the range of 1:1 to 5000:1.

8. The process of any of the preceding claims characterized in that the carbene complex (2) is (methoxyphenylcarbene)pentacarbonyltungsten(O).

9. The process of any of the preceding claims characterized in that
1-pentene is reacted to produce 4-octene,
1-hexene is reacted to produce 5-decene,
cyclooctene is reacted to produce 1,9-cyclohexadecadiene,
cis-2-pentene is reacted to produce 3-hexene,
1-decene is reacted to produce 9-octadecene,
propene is reacted to produce 2-butene,
1-tetradecene is reacted to produce 12-hexacosene,
4-penten-1-yl acetate is reacted to produce 1,8-diacetoxy-4-octene,
methyl 10-undecenoate is reacted to produce 10-eicosen-1, 20-dioate,
methyl oleate is reacted to produce 9-octadecene and dimethyl 9-octadecen-1,18-dioate,
cyclopentene is reacted with 4-penten-1-yl acetate to produce 4,9-decadien-1-yl acetate, or
methyl oleate is reacted with ethylene to produce 1-decene and methyl 9-decen-1-oate.

10. The process of any of the preceding claims characterized in that said inorganic metal compound (1) is tin tetrachloride, tin tetrabromide or germanium tetrachloride, and said catalyst composition includes as

promoter silicon tetrachloride or silicon tetrabromide in a mole ratio of silicon tetrahalide:inorganic metal compound (1) from 0.1 to 1 to 200:1.

11. The process of any of claims 1 to 9 characterized in that said inorganic metal compound (1) is tin tetrachloride, tungsten hexachloride or tungsten oxytetrachloride, and said catalyst composition includes as promoter at least one tetraorganogermanium compound of the formula $GeR^{II}_4$ wherein $R^{II}$ is an alkyl, aryl, or alkaryl radical or up to 10 carbon atoms, in a mole ratio of tetraorganogermanium compound to inorganic metal compound (1) from 0.1:1 to 100:1.

12. The process of any of claims 1 to 9 characterized in that said inorganic metal compound (1) is germanium tetrachloride or tin tetrachloride and said catalyst composition includes as promoter carbon tetrachloride in a mole ratio of carbon tetrachloride to inorganic metal compound (1) from 0.1:1 to 200:1.

13. The process of any of claims 1 to 9 characterized in that the inorganic metal compound (1) is zirconium tetrachloride and said catalyst composition includes as promoter at least one tetraorganotin compound of the formula $SnR^{II}_4$ wherein $R^{II}$ is an alkyl, aryl, or alkaryl radical of up to 10 carbon atoms, in a mole ratio of tetraorganotin compound to zirconium tetrachloride from 0.1:1 to 200:1.

14. The process of claim 13 characterized in that said tetraorganotin compound is tetramethyl tin.

15. An olefin disproportionation catalyst comprising an inorganic metal compound (1) and a neutral carbene complex (2) identified respectively as components (1) and (2) in claim 1.

16. The catalyst of claim 15, being a catalyst as hereinbefore identified in and required by any of claims 3, 4, 5 and 8.

17. The catalyst of claim 15 or 16, additionally containing a promoter and being a catalyst hereinbefore identified and required by any of claims 10—14.

18. An olefin disproportionation catalyst comprising

(1) silicon tetrachloride;

(2) at least one neutral carbene complex of the formula

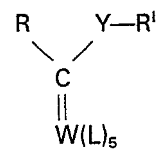

wherein R is an alkyl or cycloalkyl group of up to 10 carbon atoms or an aryl or substituted aryl radical of 6—30 carbon atoms, the substituent(s) in the latter case being selected from halogen and $C_1$—$C_{20}$ alkyl and alkoxy groups; $R^I$ and Y are as defined in claim 1; wherein R and $R^I$ are not both aryl or substituted aryl when Y is sulfur; and L is CO or NO; and

(3) carbon tetrachloride;

the molar ratio of (1):(2) being from 1:1 to 500:1 and the molar ratio of (3) to (1) being from 0.1:1 to 200:1.

19. The catalyst of claim 18 characterized in that L is CO.

20. The catalyst of claim 18 or 19 characterized in that said carbene complex (2) is (methoxyphenylcarbene)pentacarbonyltungsten(O).

21. The catalyst of any of claims 18 to 20 characterized in that the molar ratio of components (3) to (1) is in the range 1.5:1 to 50:1.

**Patentansprüche**

1. Verfahren zur Disproportionierung von Olefinen, bei dem Olefin-Reaktionspartner, wie nachstehend definiert, mit einem Disproportionierungs-Katalysator in Kontact gebracht werden, um eine Disproportionierung der Olefin-Reaktionspartner in nichtpolymere Disproportionierungsprodukte zu bewirken, dadurch gekennzeichnet, daß der Disproportionierungsprozeß in Gegenwart einer Katalysator-Zusammensetzung durchgeführt wird, die folgende Komponenten enthält:

(1) zumindest eine, aus der Gruppe $SnCl_4$, $SnBr_4$, $GeCl_4$, $TiBr_4$, $ZrCl_4$, $TaCl_5$, $MoCl_5$, $MoOCl_4$, $ReCl_5$, $FeCl_3$, $CuCl_2$, $CuBr_2$, $WCl_6$, $WOCl_4$ ausgewählte anorganische Metallverbindung; und

(2) zumindest einen neutralen Carbenkomplex der Formel

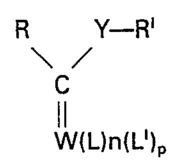

wobei

R ein Aryl- oder substituierter Arylrest mit 6 bis 30 Kohlenstoffatomen ist, dessen Substituenten im Falle eines substituierten Aryls aus Halogen-, Alkyl- und Alkoxysubstituenten mit 1 bis 20 Kohlenstoffatomen ausgewählt werden;

$R^I$ ein Alkyl-, Cycloalkyl-, Aryl-, substituierter Aryl-, Triarylsilyl- oder Trialkylsilylrest mit bis zu 30

Kohlenstoffatomen pro Rest ist, dessen Substituenten im Falle eines substituierten Aryls wie oben in Verbindung mit R definiert sind; L ein aus CO, NO, $PCl_3$, $PF_3$, Pyridin und $PR_3^I$ mit obiger Definition für $R^I$ ausgewähltes, neutrales Ligantenmolekül ist; $L^I$ ein Cyclopentadienylrest ist; p den Wert 0 oder 1 hat; n den Wert 5 hat, wenn p den Wert 0 hat, bzw. den Wert 2 hat, wenn p den Wert 1 hat; und Y Sauerstoff oder Schwefel ist;

und wobei die Olefin-Reaktionspartner 3 bis 40 Kohlenstoffatome mit einer oder mehreren nichtkonjugierten Kohlenstoff-Kohlenstoffdoppelbindungen enthalten, und wenigstens einer der Olefin-Reaktionspartner maximal zwei Kohlenstoff-Kohlenstoffdoppelbindungen enthält und entweder

(a) ein acyclisches Olefin der Formel

$$R^{III}CH{=}CHR^{IV}$$

ist, wobei $R^{III}$ und $R^{IV}$ unabhängig entweder Wasserstoff oder einen Alkyl-, Alkaryl-, oder Alkenylrest mit bis zu 18 Kohlenstoffatomen bedeuten oder

(b) ein monocyclisches Olefin der Formel

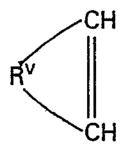

ist, wobei $R^V$ ein Alkylen- oder Alkenylenrest mit 5 bis 16 Kohlenstoffatomen ist, in welchem

— jeder der Reste $R^{III}$, $R^{IV}$ und $R^V$ als Substituenten beliebige der folgenden enthalten kann: Halogen; eine Ester- oder Ethergruppe, eine $—OSiR_3^{VI}—$ oder $—SiR_3^{VI}$-Gruppe, worin $R^{VI}$ eine Alkyl-, Aryl-, oder substituierte Arylgruppe mit bis zu 20 Kohlenstoffatomen ist, deren Substituenten im Falle eines substituierten Aryls wie in Verbindung mit R definiert sind; $—N^+R_3^{VII}X^-$, worin zumindest eine $R^{VII}$-Gruppe eine $C_1—C_{10}$-Alkylgruppe ist und die anderen Wasserstoff oder $C_1—C_{10}$-Alkyl sind, und X die Bedeutung Cl, Br oder I hat; und worin solche Substituenten im Falle von Halogen-, Ester-, Ether- und $—OSiR_3^{VI}$-Gruppen mindestens zwei Kohlenstoffatome von jeglichem doppelt gebundenem Kohlenstoffatom in dem Olefin-Reaktionspartner entfernt sind, und im Falle von $—SiR_3^{VI}—$ und $—N^+R^{VII}{}_3X^-$-Gruppen mindestens 1 Kohlenstoffatom von einem solchen doppelt gebundenen Kohlenstoffatom entfernt sind; mit der Maßgabe, daß

— $R^I$ kein Aryl oder substituiertes Aryl ist, wenn Y Schwefel ist, es sei denn, die anorganische Metallverbindung ist $SnCl_4$, $SnBr_4$ oder $GeCl_4$ und enthält als zusätzliche Komponente auch $SiCl_4$ oder $SiBr_4$ und es werden nichtfunktionelle Olefine verwendet; und

— Y immer dann Sauerstoff ist, wenn der Olefin-Reaktionspartner als Substituenten eine Estergruppe enthält; und

— die Metallkomponente des Katalysators ein Chlorid oder Oxychlorid ist, wenn das Olefin andere funktionelle Gruppen als Halogen enthält.

2. Verfahren nach Anspruch 1, dadurch gekennzeichnet, daß man die Disproportionierung bei einer Temperatur im Bereich von 35 bis 200°C und einem Druck von Atmosphärendruck bis 34,5 Mpa durchführt.

3. Verfahren nach Anspruch 1 oder 2, gekennzeichnet durch ein Molverhältnis der Komponenten (1) und (2) in dem Katalysator von 1:1 bis 500:1, wenn (1) eine Metallverbindung der Gruppe IVa ist; von 0,1:1 bis 50:1, wenn (1) eine Metallverbindung der Gruppe IVb, VIb, VIIb oder VIII ist; und von 0,1:1 bis 8:1, wenn (1) eine Metallverbindung der Gruppe Vb oder Ib ist.

4. Verfahren nach einem der vorhergehenden Ansprüche, dadurch gekennzeichnet, daß R ein Arylrest mit 6 bis 12 Kohlenstoffatomen ist; $R^I$ ein Alkylrest mit 1 bis 10 Kohlenstoffatomen ist; L die Bedeutung CO hat, n den Wert 5 hat und p den Wert 0 hat.

5. Verfahren nach Anspruch 4, dadurch gekennzeichnet, daß die anorganische Metallverbindung (1) Ti enthält und daß Y Sauerstoff ist.

6. Verfahren nach einem der vorhergehenden Ansprüche, dadurch gekennzeichnet, daß die Olefin-Reaktionspartner Kohlenwasserstoffe sind.

7. Verfahren nach einem der vorhergehenden Ansprüche, dadurch gekennzeichnet, daß das Molverhältnis von Olefin-Reaktionspartner zu Carbenkomplex (2) im Bereich von 1:1 bis 5000:1 liegt.

8. Verfahren nach einem der vorhergehenden Ansprüche, dadurch gekennzeichnet, daß der Carbenkomplex (2) (Methoxyphenylcarben)pentacarbonylwolfram(O) ist.

9. Verfahren nach einem der vorhergehenden Ansprüche, dadurch gekennzeichnet, daß

1-Penten zur Herstellung von 4-Octen,
1-Hexen zur Herstellung von 5-Decen,
Cycloocten zur Herstellung von 1,9-Cyclohexadecadien,
cis-2-Penten zur Herstellung von 3-Hexen,
1-Decen zur Herstellung von 9-Octadecen,

Propen zur Herstellung von 2-Buten,
1-Tetradecen zur Herstellung von 12-Hexacosen,
4-Penten-1-ylacetat zur Herstellung von 1,8-Diacetoxy-4-octen,
Methyl-10-undecenoat zur Herstellung von 10-Eicosen-1,20-dioat,
Methyloleat zur Herstellung von 9-Octadecen und Dimethyl-9-octadecen-1,18-Dioat,
Cyclopenten mit 4-Penten-1-ylacetat zur Herstellung von 4,9-Decadien-1-ylacetat, oder
Methyloleat mit Ethylen zur Herstellung von 1-Decen und Methyl-9-Decen-1-oat
umgesetzt wird.

10. Verfahren nach einem der vorhergehenden Ansprüche, dadurch gekennzeichnet, daß die anorganische Metallverbindung (1) Zinntetrachlorid, Zinntetrabromid oder Germaniumtetrabromid ist, und daß die Katalysatorzusammensetzung als Promotor Siliciumtetrachlorid oder Siliciumtetrabromid in einem Molverhältnis von Siliciumtetrahalogenid: anorganische Metallverbindung (1) von 0,1 zu 1 bis 200:1 enthält.

11. Verfahren nach einem der Ansprüche 1—9, dadurch gekennzeichnet, daß die anorganische Metallverbindung (1) Zinntetrachlorid, Wolframhexachlorid oder Wolframoxytetrachlorid ist, und daß die Katalysatorzusammensetzung als Promotor zumindest eine Tetraorganogermaniumverbindung der Formel $GeR^{II}_4$ in einem Molverhältnis von Tetraorganogermaniumverbindung zu anorganischer Metallverbindung (1) von 0,1:10 bis 100:1 enthält, wobei $R^{II}$ ein Alkyl-, Aryl- oder Alkarylrest mit bis zu 10 Kohlenstoffatomen ist.

12. Verfahren nach einem der Ansprüche 1—9, dadurch gekennzeichnet, daß die anorganische Metallverbindung (1) Germaniumtetrachlorid oder Zinntetrachlorid ist, und daß die Katalysatorzusammensetzung als Promotor Kohlenstofftetrachlorid in einem Molverhältnis von Kohlenstofftetrachlorid zu anorganischer Metallverbindung (1) von 0,1:1 bis 200:1 enthält.

13. Verfahren nach einem der Ansprüche 1—9, dadurch gekennzeichnet, daß die anorganische Metallverbindung (1) Zirconiumtetrachlorid ist, und daß die Katalysatorzusammensetzung als Promotor zumindest eine Tetraorganozinnverbindung der Formel $SnR_4^{II}$ in einem Molverhältnis von Tetraorganozinnverbindung zu Zirconiumtetrachlorid von 0,1:1 bis 200:1 enthält, wobei $R^{II}$ ein Alkyl-, Aryl- oder Alkarylrest mit bis zu 10 Kohlenstoffatomen ist.

14. Verfahren nach Anspruch 13, dadurch gekennzeichnet, daß die Tetraorganozinnverbindung Tetramethylzinn ist.

15. Katalysator zu Olefin-Disproportionierung, der eine anorganische Metallverbindung (1) und einen neutralen Carbenkomplex (2), in Anspruch 1 als Komponenten (1) und (2) definiert, enthält.

16. Katalysator nach Anspruch 15, weiterhin gekennzeichnet durch die Erfordernisse von einem der Ansprüche 3, 4, 5 und 8.

17. Katalysator nach Anspruch 15 oder 16, der zusätzlich einen Promotor enthält und die Erfordernisse von einem der Ansprüche 10—14 erfüllt.

18. Katalysator zur Olefin-Disproportionierung, der
(1) Siliciumtetrachlorid;
(2) zumindest einen neutralen Carbenkomplex der Formel

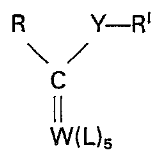

wobei R eine Alkyl- oder Cycloalkylgruppe mit bis zu 10 Kohlenstoffatomen oder ein Aryl- oder substituierten Arylrest mit 6 bis 30 Kohlenstoffatomen ist, und der (die) Substituent(en) in letzterem Falle aus Halogen-, $C_1$ bis $C_{20}$-Alkyl- und Alkoxygruppen ausgewählt werden; $R^I$ und Y wie in Anspruch 1 definiert sind, wobei R und $R^I$ nicht beide Aryl- oder substituierte Arylgruppen sind, wenn Y Schwefel ist; und L die Bedeutung CO oder NO hat; und
(3) Kohlenstofftetrachlorid;
enthält, wobei das Molverhältnis von (1) zu (2) von 1:1 bis 500:1 und das Molverhältnis von (3) zu (2) von 0,1:1 bis 200:1 reicht.

19. Katalysator nach Anspruch 18, dadurch gekennzeichnet, daß L die Bedeutung CO hat.

20. Katalysator nach Anspruch 18 oder 19, dadurch gekennzeichnet, daß der Carbenkomplex (2) (Methoxyphenylcarben)pentacarbonylwolfram(O) ist.

21. Katalysator nach einem der Ansprüche 18 bis 20, dadurch gekennzeichnet, daß das Molverhältnis der Komponenten (3) zu (1) im Bereich von 1,5:1 bis 50:1 liegt.

**Revendications**

1. Procédé de dismutation des oléfines qui consiste à mettre en contact des réactifs oléfiniques, tels que définis ci-après, avec un catalyseur de dismutation pour provoquer une dismutation des reactifs

oléfiniques en produits de dismutation non polymères, caractérisé en ce qu'on effectue ce procédé de dismutation en présence d'une composition de catalyseurs comprenant:

(1) au moins un composé métallique minéral choisi parmi $SnCl_4$, $SnBr_4$, $GeCl_4$, $TiBr_4$, $ZrCl_4$, $TaCl_5$, $MoCl_5$, $MoOCl_4$, $ReCl_5$, $FeCl_3$, $CuCl_2$, $CuBr_2$, $WCl_6$ et $WOCl_4$; et

(2) au moins un complexe de carbène neutre répondant à la formule:

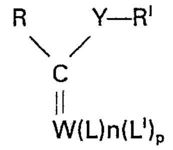

dans laquelle

R est un radical aryle ou aryle substitué en $C_6$ à $C_{30}$, les substituants dans le cas de l'aryle substitué étant choisis parmi un halogène et des radicaux alkyle et alcoxy en $C_1$ à $C_{20}$,

$R^I$ est un radical alkyle, cycloalkyle, aryle, aryle substitué, triarylsilyle ou trialkylsilyle contenant jusqu'à 30 atomes de carbone par radical, les substituants dans le cas de l'aryle substitué étant tels que définis ci-dessus à propos de R; L est une molécule de coordinat neutre choisie parmi CO, NO, $PCl_3$, $PF_3$, la pyridine et $PR^I_3$ où $R^I$ est tel que défini ci-dessus;

$L^I$ est un radical cyclopentadiényle; p est 0 ou 1; n est 5 lorsque p est 0, ou 2 lorsque p est 1; et Y est l'oxygène ou le soufre,

et dans laquelle ces réactifs oléfiniques contiennent de 3 à 40 atomes de carbone et une ou plusieurs doubles liaisons carbone-carbone non conjuguées et au moins un de ces réactifs oléfiniques contient un maximum de deux doubles liaisons carbone-carbone et est soit:

(a) une oléfine acyclique répondant à la formule:

$$R^{III}CH{=}CHR^{IV}$$

dans laquelle $R^{III}$ et $R^{IV}$ sont indépendamment l'hydrogène ou un radical alkyle, alcaryle ou alcényle ayant jusqu'à 18 atomes de carbone, ou

(b) une oléfine monocyclique répondant à la formule:

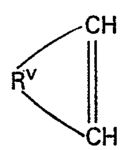

dans laquelle $R^V$ est un radical alkylène ou alcénylène contenant de 5 à 16 atomes de carbone, dans lequel:

— chacun des radicaux $R^{III}$ et $R^{IV}$ et $R^V$ peut contenir comme substituant n'importe lesquels des suivants: un halogène; un groupe ester ou éther; un groupe $-OSiR^{VI}_3$ ou $-SiR^{VI}_3$ dans lequel $R^{VI}$ est un groupe alkyle, aryle ou aryle substitué ayant jusqu'à 20 atomes de carbone, les substituants dans le cas de l'aryle substitué étant tels que définis ci-dessus à propos de R; $-N^+R_3^{VII}X^-$ où au moins un groupe $R^{VII}$ est un groupe alkyle en $C_1$ à $C_{10}$ et les autres sont l'hydrogène ou un groupe alkyle en $C_1$ à $C_{10}$, et X est Cl, Br ou I; ces substituants dans le cas des groupes halo, ester, éther et $-OSiR^{VI}_3$ étant éloignés d'au moins deux atomes de carbone de n'importe quel atome de carbone doublement lié du réactif oléfinique et, dans le cas de groupes $-SiR^{VI}_3$ et $-N^+-R^{VII}{}_3X^-$ éloignés d'au moins un atome de carbone de n'importe lequel de ces atomes de carbone doublement liés, sous les réserves que

— $R^I$ soit autre qu'un radical aryle ou aryle substitué lorsque Y est le soufre, à moins que ce composé métallique minéral ne soit $SnCl_4$, $SnBr_4$ ou $GeCl_4$ et contienne aussi $SiCl_4$ ou $SiBr_4$ comme constituant supplémentaire, et que l'on utilise des oléfines non fonctionnelles; et

— Y soit l'oxygène toutes les fois que le réactif oléfinique contient un groupe ester comme substituant; et

— le constituant métallique du catalyseur soit un chlorure ou un oxychlorure lorsque l'oléfine contient des groupes fonctionnels autres que des halogènes.

2. Procédé selon la revendication 1, caractérisé en ce que cette dismutation est effectuée à une température dans l'intervalle de 35 à 200°C et sous une pression allant de la pression atmosphérique à 34,5 MPa.

3. Procédé selon les revendications 1 ou 2, caractérisé en ce que le rapport molaire des constituants (1) et (2) dans le catalyseur est de 1:1 à 500:1, lorsque (1) est un composé d'un métal du groupe IVa; de 0,1:1 à 50:1 lorsque (1) est un composé d'un métal des groupes IVb, VIb, VIIb, ou VIII; et de 0,1:1 à 8:1 lorsque (1) est un composé de métaux des groupes Vb ou Ib.

4. Procédé selon l'une quelconque des revendications précédentes, caractérisé en ce que R est un radical aryle contenant 6 à 12 atomes de carbone, $R^I$ est un radical alkyle contenant de 1 à 10 atomes de carbone; L est CO, n est 5 et p est 0.

5. Procédé selon la revendication 4, caractérisé en ce que ce composé métallique minéral (1) contient du Ti et en ce que Y est l'oxygène.

6. Procédé selon l'une quelconque des revendications précédentes, caractérisé en ce que les réactifs oléfiniques sont des hydrocarbures.

7. Procédé selon l'une quelconque des revendications précédentes, caractérisé en ce que le rapport molaire du réactif oléfinique au complexe de carbène (2) est dans l'intervalle de 1:1 à 5000:1.

8. Procédé selon l'une quelconque des revendications précédentes, caractérisé en ce que le complexe de carbène (2) est du (méthoxyphénylcarbène)pentacarbonyltungstène(O).

9. Procédé selon l'une quelconque des revendications précédentes, caractérisé en ce que:
on fait réagir du 1-pentène pour produire du 4-octène,
on fait réagir du 1-hexène pour produire du 5-décène,
on fait réagir du cyclooctène pour produire du 1,9-cyclohexadécadiène,
on fait réagir du cis-2-pentène pour produire du 3-hexène,
on fait réagir du 1-décène pour produire du 9-octadécène,
on fait réagir du propène pour produire du 2-butène,
on fait réagir du 1-tétradécène pour produire du 12-hexacosène,
on fait réagir de l'acétate de 4-pentén-1-yle pour produire du 1,8-diacétoxy-4-octène,
on fait réagir du 10-undécénoate de méthyle pour produire du 10-eicosèn-1,20-dioate,
on fait réagir de l'oléate de méthyle pour produire du 9-octadécène et du 9-octadécène-1,18-dioate de diméthyle,
on fait réagir du cyclopentène avec de l'acétate de 4-pentén-1-yle pour produire de l'acétate de 4,9-décadién-1-yle, ou
on fait réagir de l'oléate de méthyle avec de l'éthylène pour produire du 1-décène et du 9-décène-1-oate de méthyle.

10. Procédé selon l'une quelconque des revendications précédentes, caractérisé en ce que ce composé métallique minéral (1) est du tétrachlorure d'étain, du tétrabromure d'étain ou du tétrachlorure de germanium et en ce que cette composition de catalyseur contient comme promorteur du tétrachlorure de silicium ou du tétrabromure de silicium dans un rapport molaire tétrahalogénure de silicium: composé métallique minéral (1) de 0,1 à 1 à 200:1.

11. Procédé selon l'une quelconque des revendications 1 à 9, caractérisé en ce que ce composé métallique minéral (1) est le tétrachlorure d'étain, l'hexachlorure de tungstène ou l'oxytétrachlorure de tungstène et en ce que cette composition de catalyseurs contient comme promoteur au moins un composé tétraorganogermanium répondant à la formule $GeR^{II}$ où $R^{II}$ est un radical alkyle, aryle ou alkcaryle ayant jusqu'à 10 atomes de carbone, dans un rapport molaire du composé du tétraorganogermanium ou composé métallique minéral (1) de 0,1:1 à 100:1.

12. Procédé selon l'une quelconque des revendications 1 à 9, caractérisé en ce que ce composé métallique minéral (1) est du tétrachlorure de germanium ou du tétrachlorure d'étain et en ce que cette composition de catalyseurs contient comme promoteur du tétrachlorure de carbone dans un rapport molaire du tétrachlorure de carbone au composé métallique minéral (1) de 0,1:1 à 200:1.

13. Procédé selon l'une quelconque des revendications 1 à 9, caractérisé en ce que le composé métallique minéral (1) est du tétrachlorure de zirconium et en ce que cette composition de catalyseurs contient comme promoteur au moins un composé tétraorganostannique répondant à la formule $SnR^{II}_4$ où $R^{II}$ est un radical alkyle, aryle ou alcaryle ayant jusqu'à 10 atomes de carbone, dans un rapport molaire du composé tétraorganostannique au tétrachlorure de zirconium de 0,1:1 à 200:1.

14. Procédé selon la revendication 13, caractérisé en ce que le composé tétraorganostannique est le tétraméthylétain.

15. Catalyseur de dismutation des oléfines comprenant un composé métallique minéral (1) et un complexe neutre du carbène (2), identifiés respectivement comme constituants (1) et (2) dans la revendication 1.

16. Catalyseur selon la revendication 15, qui est un catalyseur tel qu'identifié précédemment dans l'une quelconque des revendications 3, 4, 5 et 8 et exigé par celles-ci.

17. Catalyseur selon les revendications 15 ou 16, contenant en outre un promoteur et qui est un catalyseur précédemment identifié par l'une quelconque des revendications 10—14 et exigé par celles-ci.

18. Catalyseur de dismutation des oléfines comprenant:
(1) du tétrachlorure de silicium;
(2) au moins un complexe de carbène neutre répondant à la formule:

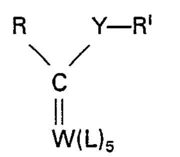

dans laquelle R est un groupe alkyle ou cycloalkyle ayant jusqu'à 10 atomes de carbone et un radical aryle ou aryle substitué ayant de 6 à 30 atomes de carbone, le ou les substituant(s) dans ce denier cas étant

choisis parmi un halogène et des groupes alkyle et alcoxy en $C_1$ à $C_{20}$; $R^I$ et Y sont tels que définis dans la revendication 1; dans laquelle R et $R^I$ ne sont pas tous les deux des groupes aryle ou aryle substitué lorsque Y est le soufre; et L est CO ou NO; et

(3) du tétrachlorure de carbone;

le rapport molaire de (1):(2) étant de 1:1 à 500:1 et le rapport molaire de (3) à (1) étant de 0,1:1 à 200:1.

19. Catalyseur selon la revendication 18, caractérisé en ce que L est CO.

20. Catalyseur selon les revendications 18 ou 19, caractérisé en ce que ce complexe de carbène (2) est le (méthoxyphénylcarbène)pentacarbonyltungstène(O).

21. Catalyseur selon l'une quelconque des revendications 18 à 20, caractérisé en ce que le rapport molaire des constituants (3) à (1) est dans l'intervalle de 1,5:1 à 50:1.